# EUROPÄISCHE PATENTSCHRIFT

(11) **EP 3 573 534 B1**
(45) Veröffentlichungstag und Bekanntmachung des Hinweises auf die Patenterteilung: **30.06.2021**
(21) Anmeldenummer: 18706961.2
(22) Anmeldetag: 29.01.2018
(51) Int. Cl.: A61B 8/08, A61B 34/20, A61B 34/30, A61B 5/15, A61B 34/32, A61B 5/00, A61B 90/14, A61B 90/00, A61M 1/36, A61M 5/42

(54) **VORRICHTUNG ZUR ERKENNUNG UND MANIPULATION EINES BLUTGEFÄSSES, SOWIE ENTSPRECHENDES VERFAHREN**
APPARATUS FOR IDENTIFYING AND MANIPULATING A BLOOD VESSEL, AND CORRESPONDING METHOD
DISPOSITIF POUR DÉTECTER ET MANIPULER UN VAISSEAU SANGUIN, ET PROCÉDÉ CORRESPONDANT

(30) Priorität: 30.01.2017 DE 102017201440
(43) Veröffentlichungstag der Anmeldung: 04.12.2019
(73) Patentinhaber: Fresenius Medical Care Deutschland GmbH, 61352 Bad Homburg (DE)
(72) Erfinder: KOPPERSCHMIDT, Pascal, 97456 Dittelbrunn (DE); DANIEL, Pia, 78351 Bodman (DE); SPICKERMANN, Reiner, 97535 Wasserlosen-Burghausen (DE); ARKOSSY, Otto, 1037 Budapest (HU); SCHOLZ, Cäcilia, 65824 Schwalbach (DE); RITTER, Kai-Uwe, 91126 Rednitz-Hembach (DE); SCHULTE, Elke, 97422 Schweinfurt (DE); HAUKE, Christopher, 55246 Mainz-Kostheim (DE)
(74) Vertreter: Ricker, Mathias
(86) Internationale Anmeldenummer: PCT/EP2018/052147
(87) Internationale Veröffentlichungsnummer: WO 2018/138342

(56) Entgegenhaltungen:
- WO-A1-2016/087123
- FR-A1- 3 019 724
- US-A1- 2004 171 923
- US-A1- 2009 234 261
- US-A1- 2015 065 916
- US-B1- 8 888 714

## Beschreibung

Die vorliegende Erfindung liegt auf dem Gebiet der Medizintechnik und betrifft insbesondere eine Erkennungsvorrichtung zur Erkennung und Manipulation eines Blutgefäßes unter der Haut eines Körperteils eines Patienten, einen Kanülierautomaten mit einer Erkennungsvorrichtung und ein entsprechendes Verfahren zum automatisierten Erkennen und Manipulieren eines Blutgefäßes unter der Haut eines Körperteils eines Patienten.

Die Punktion von Blutgefäßen, auch bezeichnet als Kanülierung, ist ein routinemäßig erforderlicher Arbeitsschritt der medizinischen Behandlung vieler Patienten, bei dem eine fluide Verbindung zwischen dem Blutkreislauf der Patienten und einem externen Fluidsystem, insbesondere einer Kanüle, geschaffen wird. Üblicherweise wird die Kanülierung von Ärzten oder trainierten Personal durchgeführt. Die Qualität des durch die Kanülierung geschaffenen Gefäßzugangs hängt dabei von einer Vielzahl von Parametern ab, die insbesondere durch die individuellen, zeitlich veränderlichen Fähigkeiten des medizinischen Personals und körperlichen Eigenschaften der zu behandelnden Patienten sowie durch die Diversität der bei der Punktion eingesetzten technischen Hilfsmittel geprägt werden.

Auch wird eine Kanülierung, da sie ein routinemäßiger Arbeitsschritt bei vielen Behandlungen ist, häufig durchgeführt. Um dabei die Kanülierung zu vereinheitlichen, finanzielle sowie personelle Ressourcen effizient zu nutzen und eine hohe Behandlungsqualität verlässlich sicherzustellen, wurden Kanülierautomaten entwickelt, die einen Kanüliervorgang am Patienten durch geeignete Sensorik und Motorik autonom durchführen. Solche Kanülierautomaten und dabei verwendete technische Mittel sind zum Beispiel bekannt aus der EP 0 654 244 B1, der US 2015/0065916 A1 und der WO 2015/052719 A1. Erkennungsvorrichtungen zur Erkennung von Gefäßstrukturen sind an solchen Automaten ebenfalls bekannt. FR 3 019 724 A1 und US 8 888 714 B1 offenbaren eine Erkennungsvorrichtung und ein Verfahren gemäß dem Oberbegriff der unabhängigen Ansprüche 1 und 11.

Der Erfindung liegt die Aufgabe zu Grunde, insbesondere für eine weitergehende Automatisierung der Kanülierung, ein verbessertes Erkennungssystem anzugeben, mit dem eine der Erkennung nachfolgende Behandlung des Blutgefäßes effizienter ist. Die Erfindung löst diese Aufgabe jeweils durch eine Erkennungsvorrichtung gemäß der Lehre des unabhängigen Anspruchs 1 und ein Verfahren gemäß der Lehre des unabhängigen Anspruchs 11. Bevorzugte Ausführungsformen, Weiterbildungen oder Varianten sind insbesondere Gegenstand der abhängigen Ansprüche. Die Gegenstände der Ansprüche werden ausdrücklich zum Teil der Offenbarung der Beschreibung gemacht.

Die erfindungsgemäße Erkennungsvorrichtung bietet den Vorteil, dass eine anfänglich erkannte Lage und/oder Abmessungen des Blutgefäßes durch Manipulation des Blutgefäßes in einer gewünschten Weise beeinflusst und damit optimiert werden kann. Insbesondere kann ein aus physiologischen oder pathologischen Gründen verändertes Blutgefäß in der Weise manipuliert werden, dass eine nachfolgende Behandlung, insbesondere automatische Kanülierung möglich ist.

Die Gefäßstrukturmesseinrichtung ist zur Erkennung der Lage und/oder Abmessungen des Blutgefäßes durch Messung von Gefäßstrukturdaten des Blutgefäßes im Behandlungsraum eingerichtet. Die Messung der Lage und/oder Abmessungen des Blutgefäßes in Form von Gefäßstrukturdaten und die nachfolgende Manipulation dieser Lage und/oder Abmessungen mittels der Gefäßmanipulationseinrichtung dienen vorzugsweise der Vorbereitung einer möglichen anschließenden Behandlung des Patienten, insbesondere der nachfolgenden automatischen Kanülierung des Blutgefäßes. Die Manipulation dient hierbei insbesondere als korrigierende Maßnahme, um eine gewünschte Verbesserung der Lage und/oder Abmessungen des Blutgefäßes herbeizuführen.

Die Gefäßstrukturdaten werden insbesondere dahingehend ausgewertet, ob die Lage und/oder Abmessungen des Blutgefäßes mindestens einem Kriterium entsprechen. Dieses Kriterium kann insbesondere für die nachfolgende Behandlung des Patienten von Bedeutung sein, insbesondere die Frage, ob eine solche Behandlung durchführbar ist und/oder in welcher Modifikation diese Behandlung durchzuführen ist. Durch Manipulation des Blutgefäßes mit der Gefäßmanipulationseinrichtung werden die Lage und/oder die Abmessung verändert, und das Kriterium wird nach Durchführung der Manipulation erneut geprüft. Die Erfindung ist besonders in solchen Fällen vorteilhaft, in denen das Blutgefäß in der anfänglichen Lage und/oder der anfänglichen Abmessung -also insbesondere vor der Manipulation- das mindestens eine Kriterium nicht erfüllt hat und die nachfolgende Manipulation dem Zweck dient, dieses Kriterium nachfolgend zu erfüllen. Die Manipulation kann beispielsweise eine Blutstauung im Blutgefäß mittels einer Blutstauungseinrichtung vorsehen, die zu einem Anschwellen des Blutgefäßes führt, und zu einer vorbestimmten Dicke des Blutgefäßes führt, die ausreichend ist, um eine automatische Kanülierung zuverlässig durchführen zu können.

Es wird vorliegend meist auf "das" Blutgefäß, also den Singular Bezug genommen. Die Erfindung bezieht sich aber genauso auf die Erkennung und Manipulation von mehr als einem Blutgefäß.

Die Gefäßstrukturdaten, welche die Informationen über die Lage eines Blutgefäßes enthalten, können die Positionsdaten enthalten, mit dem sich der Raumbereich oder eine Veränderung des Raumbereichs eindeutig ermitteln lässt, der von diesem Raumbereich eingenommen wird. Diese Positionsdaten können zu mindestens einem festen Bezugspunkt der Erkennungsvorrichtung definiert sein, oder einem festen Bezugspunkt der Behandlungsvorrichtung, insbesondere des Kanülierautomaten, die die Erkennungsvorrichtung optional aufweist. Alternativ kommt auch in Betracht, mit diesen Gefäßstrukturdaten die Lage in der Weise zu erfassen, dass nur die Positionsdaten der Hülle des Blutgefäßes gespeichert werden, also beispielsweise der Verlauf der Blutgefäßwand, die das Blut kanalisiert. Weiter alternativ, oder zusätzlich, kommt auch in Betracht, mit diesen Gefäßstrukturdaten die Lage in der Weise zu erfassen, dass die Positionsdaten des vom fließenden Blut durchströmten Raumbereichs erfasst werden, was beispielsweise im Fall von Erfassung der Positionsdaten mit Ultraschallmessungen im Dopplerverfahren möglich ist. Die Positionsdaten können so auch alternativ oder zusätzlich als Geschwindigkeitsdaten gespeichert werden.

Anstelle -oder zusätzlich- zu solchen absoluten Positionsdaten können die Positionsdaten die Veränderungen der Lage des Blutgefäßes als relative Positionsdaten enthalten.

Die Gefäßstrukturdaten, welche die Informationen über die Abmessungen eines Blutgefäßes enthalten, können insbesondere Informationen über die Dicke des Blutgefäßes, oder die Dicke des vom Blut durchflossenen Raumbereichs, gemessen senkrecht zur Fließrichtung, also Längsrichtung, des Blutgefäßes enthalten, insbesondere gemessen parallel zur Hautoberfläche des Körperteils des Patienten, und/oder insbesondere gemessen senkrecht zur Hautoberfläche des Körperteils des Patienten.

Die Gefäßstrukturdaten können auch Näherungsdaten der Lage und/oder Abmessungen des Blutgefäßes enthalten, indem beispielsweise die Erfassung von Positionsdaten oder Distanzdaten nur abschnittsweise erfolgt, und/oder mit einer reduzierten Auflösung, während die Lage und/oder Abmessungen des Blutgefäßes in der erforderlichen Genauigkeit geschätzt und mittels Modellanwendung ergänzt werden, z.B. durch ein Interpolationsverfahren.

Die Gefäßstrukturmesseinrichtung kann als Bilderfassungseinrichtung eingerichtet sein, so dass Bilddaten des mindestens einen Blutgefäßes, vorzugsweise eines Verbundes aus mehreren Blutgefäßen, als Gefäßstrukturdaten erfasst werden. Die Gefäßstrukturdaten werden vorzugsweise in einer Datenspeichereinrichtung, die Teil der Erkennungsvorrichtung sein kann, abgespeichert. Dadurch stehen die Gefäßstrukturdaten für die nachfolgende Auswertung zur Verfügung und können optional auch permanent gespeichert werden, insbesondere einem Patienten als Patientendaten zugeordnet werden und für eine spätere Verwendung im Verlauf beispielsweise einer längeren Behandlung, insbesondere bei chronischer Erkrankung, erneut herangezogen werden.

Die Gefäßstrukturmesseinrichtung, insbesondere die Bilderfassungseinrichtung, kann für eine optische Messung eingerichtet sein. Als Bild wird insbesondere ein Datensatz bezeichnet, der die Information über die Lage und/oder räumlichen Abmessungen des mindestens einen Blutgefäßes enthält. Dabei weist die Bilderfassungseinrichtung vorzugsweise einen oder mehrere optische Sensoren oder mindestens eine Kamera auf, mittels derer im Wellenlängenbereich des sichtbaren Lichts, d.h. zwischen 380 und 780 nm, eine oder mehrere Bildaufnahmen erstellbar und digital speicherbar sind. Die Gefäßstrukturmesseinrichtung, insbesondere die Bilderfassungseinrichtung, kann insbesondere auch zur Messung in anderen Bereichen des elektromagnetischen Spektrums eingerichtet sein, insbesondere im ultravioletten Bereich, also zwischen 100 nm und 380 nm, oder im Infrarotbereich, also zwischen 780 nm und 1 mm, vorzugsweise zusätzlich oder alternativ zur Messung im sichtbaren Spektrum. Dadurch kann der Kontrast angepasst werden. Die optische Erfassung von Blutgefäßen wird zum Beispiel in WO 2010/029521 A2 beschrieben.

Alternativ kann die Gefäßstrukturmesseinrichtung, insbesondere die Bilderfassungseinrichtung, für eine Messung mittels Computertomographie (CT), Magnetresonanztomographie (MRT), oder Positronen-Emissions-Tomographie (PET) ausgebildet sein.

Die Gefäßstrukturmesseinrichtung, insbesondere die Bilderfassungseinrichtung, ist vorzugsweise für eine -insbesondere sonographische- Messung mittels Ultraschall eingerichtet, insbesondere für Messungen im Duplexverfahren bzw. im Dopplerverfahren. Dadurch kann die Bilderfassung mit relativ geringem apparativem Aufwand erfolgen. Die Erfassung von Blutgefäßen mittels Ultraschall ist bewährt und wird zum Beispiel in US 2008/0146939 A1 beschrieben.

Vorzugsweise ist die Gefäßstrukturmesseinrichtung, insbesondere die Bilderfassungseinrichtung, zur wiederholten Messung der Lage und/oder Abmessungen des Blutgefäßes eingerichtet, insbesondere zur phasenweisen oder kontinuierlich wiederholten Messung, insbesondere mit vorgegebener zeitlicher Frequenz. Eine Frequenz von beispielsweise einer Messung pro Sekunde (1 Hz) oder größer erlaubt eine genaue Erfassung der Lage und/oder Abmessungen des Blutgefäßes und damit eine bessere Kontrolle für die nachfolgende Gefäßmanipulation, so dass eine optional nachfolgende Behandlung des Patienten, insbesondere eine automatische Kanülierung des Blutgefäßes, genauer durchführbar und kontrollierbar ist.

Die Erkennungsvorrichtung weist vorzugsweise eine Basis auf, auf der vorzugsweise alle Bestandteile der Erkennungsvorrichtung montiert sind, insbesondere die datenverarbeitende Steuerungseinrichtung, die Gefäßstrukturmesseinrichtung und die Gefäßmanipulationseinrichtung. Falls die Erkennungsvorrichtung ein Bestandteil einer Behandlungsvorrichtung ist, z.B. eines Kanülierautomaten, kann die Basis der Erkennungsvorrichtung ein Bestandteil der Behandlungsvorrichtung sein.

Der Behandlungsraum kann ein teilweise umhüllter oder freier Raumbereich sein, der insbesondere in eine Behandlungsvorrichtung integriert sein kann, insbesondere in einen Kanülierautomaten. Der Behandlungsraum dient dazu, das Körperteil des Patienten zumindest teilweise aufzunehmen, das das zu erkennende Blutgefäß unter der Haut aufweist. Das Körperteil ist vorzugsweise ein Arm oder ein Bein.

Der Behandlungsraum weist vorzugsweise eine Stützeinrichtung zur Abstützung des Körperteils auf, insbesondere eine Auflage, oder mehrere Auflagen.

Der Behandlungsraum kann eine Fixiereinrichtung aufweisen, mittels der das Körperteil relativ zum Behandlungsraum oder relativ zu der Stützeinrichtung fixierbar ist und bei der Erkennung und/oder nachfolgenden Behandlung, insbesondere Kanülierung, fixiert wird. Die Fixiereinrichtung kann mindestens ein Fixierband aufweisen, mittels dem das Körperteil an der Stützeinrichtung fixiert wird. Die Fixiereinrichtung kann ferner auch die Funktion der Stützeinrichtung übernehmen, indem beispielsweise das Körperteil mit der Fixiereinrichtung im Behandlungsraum aufgehängt wird. Die Fixierung erfolgt vorzugsweise so, dass die translatorische und/oder rotatorische Beweglichkeit des Körperteils in wenigstens einer, wenigsten zwei, drei, vier, fünf, sechs Raumrichtungen eingeschränkt wird, vorzugsweise in allen sechs Raumrichtungen in positiver und negativer Richtung entlang der drei orthogonalen Raumachsen eines kartesischen Koordinatensystems und/oder allen sechs rotatorischen Richtungen um diese Raumachsen.

Die Fixiereinrichtung kann eine Kisseneinrichtung aufweisen oder aus dieser gebildet werden. Die Kisseneinrichtung kann zur Aufnahme eines Fluids, insbesondere Luft, Flüssigkeit oder ein Gel, ausgebildet sein. Vorzugsweise kann eine elektrisch ansteuerbare Fluidtransporteinrichtung, insbesondere Pumpe oder eine Presseinrichtung vorgesehen sein, um das Fluid zu transportieren. Dieser Transport wird vorzugsweise von der Steuerungseinrichtung automatisch gesteuert, so dass die Fixierung vorzugsweise automatisch von der Erkennungsvorrichtung durchgeführt werden kann. Zum Fixieren kann vorgesehen sein, dass das Fluidvolumen in der Kisseneinrichtung mittels der Fluidtransporteinrichtung erhöht wird, so dass die Beweglichkeit des an der Kisseneinrichtung angeordneten Körperteils eingeschränkt und dieses vorzugsweise fixiert wird. Die Kisseneinrichtung kann das Körperteil teilweise oder vollständig umgeben, insbesondere kann die Kisseneinrichtung als geschlossener oder offener Schlauchring gebildet sein, ähnlich einer Armmanschette bei der Blutdruckmessung. Dabei ist die als Stütz- oder Fixiereinrichtung dienende Kisseneinrichtung vorzugsweise in der Erkennungsvorrichtung fixiert.

Die Stützeinrichtung und/oder die Fixiereinrichtung kann insbesondere zusätzlich als Gefäßmanipulationseinrichtung eingerichtet sein.

Die Gefäßmanipulationseinrichtung ist zur Änderung von Lage und/oder Abmessung des Blutgefäßes eingerichtet. Vorzugsweise ist die Gefäßmanipulationseinrichtung als Anpresseinrichtung ausgebildet, mittels der ein Druck auf das im Behandlungsraum angeordnete Körperteil ausgeübt wird, um das Blut in dem zu erkennenden Blutgefäß zu stauen. Durch das Stauen des Blutes kann gemäß dem vorbestimmten Kriterium eine bestimmte Dicke des Blutgefäßes erreicht werden und eingestellt werden. Insbesondere kann die Dicke des Blutgefäßes durch die Steuerungseinrichtung geregelt werden.

Vorzugsweise weist die Anpresseinrichtung eine elektrisch ansteuerbare Kisseneinrichtung auf, wie oben geschildert, deren Füllvolumen und damit der Anpressdruck von der Steuerungseinrichtung elektrisch gesteuert werden. Die Steuerung der Gefäßmanipulationseinrichtung, insbesondere der Anpresseinrichtung oder Kisseneinrichtung, insbesondere die Übertragung von Steuersignalen, sowie deren Versorgung mit elektrischer Energie können drahtgebunden sein, wobei ein elektrisches Kabel, insbesondere Datenübertragungskabel, dem Signalaustausch zwischen der Steuerungseinrichtung der Erkennungsvorrichtung und der Anpresseinrichtung dienen können. Der Signalaustausch kann auch drahtlos erfolgen, wobei die Anpresseinrichtung in diesem Fall als von der Erkennungsvorrichtung separates Bauteil ausgeführt sein kann. Dessen Energieversorgung kann über eine in oder an der Gefäßmanipulationseinrichtung angeordnete Batterie erfolgen. Durch die separate Ausbildung der Gefäßmanipulationseinrichtung kann der Behandlungsraum für andere Einrichtungen freigehalten werden. Zudem ist eine flexiblere Anpassung an unterschiedliche Körperteile und an unterschiedlich körperlich gebaute Patienten möglich. Die Kisseneinrichtung kann ferner eine Aktuatoreinrichtung aufweisen, mittels der eine Zugspannung auf die Kisseneinrichtung aufbringbar ist, indem beispielsweise ein Schlauchring in Umfangsrichtung angespannt wird, in dieser Weise um das Körperteil gezurrt wird und eine als Anpressdruck wirkende radial auf das Körperteil wirkenden Kraft aufbringt.

Die Gefäßmanipulationseinrichtung kann eine Temperiereinrichtung aufweisen, um den mit der Haut in Kontakt stehenden Bereich einer Anpresseinrichtung oder vorzugsweise das in einer Kisseneinrichtung enthaltene fluide Medium auf eine Solltemperatur zu erwärmen oder zu kühlen, insbesondere aufweisend einen Temperatursensor, und insbesondere zu geregelten Einstellung der Temperatur. Durch Wärme können Kapillaren geöffnet werden, wodurch die Durchblutung der Haut verbessert wird.

Die Anpresseinrichtung kann ferner ein oder mehrere Bandeinrichtungen aufweisen, die - ähnlich einem Schlauchring, aber ohne fluidgefüllten Hohlraum- in Umfangsrichtung mittels einer oder mehrerer Aktuatoreinrichtungen der Gefäßmanipulationseinrichtung angespannt werden, wobei diese insbesondere um das Körperteil gezurrt werden und eine als Anpressdruck wirkende radial auf das Körperteil wirkenden Kraft aufbringen.

Die Anpresseinrichtung kann ferner ein oder mehrere bewegliche Klammerarme aufweisen, die im Behandlungsraum beweglich montiert sein können, insbesondere an der Stützeinrichtung oder Fixiereinrichtung beweglich montiert sein können. Mittels einer oder mehrerer Aktuatoreinrichtungen der Gefäßmanipulationseinrichtung können die einen oder mehrere bewegliche Klammerarme vorzugsweise ausgelenkt werden, um den gewünschten Anpressdruck auf das Körperteil aufzubringen. Dabei kann der Gewebeabschnitt, der das Blutgefäß enthält, zwischen den Enden der Halteklammern vom Körperteil weg radial nach außen gepresst werden, wodurch einerseits die gewünschte Blutstauung erreicht wird und andererseits das Blutgefäß zugänglich ist und zusätzlich fixiert wird.

Zudem kann die die mindestens eine Halteklammer aufweisende Gefäßmanipulationseinrichtung dazu eingerichtet sein, die Haut, unter der sich das zu erkennende Blutgefäß befindet, mit einer Zugspannung zu beaufschlagen, indem Klammerame adhäsive Kontaktpunkte aufweisen, mittels denen die Haut kontaktiert und adhäsiv gehalten wird, so dass eine Zugbewegung der Halteklammern die zwischen den Kontaktabschnitten der Halteklammern liegende Haut strafft. Dadurch kann einerseits das zu erkennende Blutgefäß in der Haut fixiert werden. Andererseits kann dessen Lage durch Bewegen der an der Haut adhärierten Halteklammern manipuliert werden. Die Kontaktabschnitte können die Adhäsion insbesondere mittels eines am Kontaktabschnitt angebrachten reibungserzeugenden Materials, z.B. Silikonelastomer, oder mittels eines am Kontaktabschnitt angebrachten Klebstoffabschnitts bewirken.

Die Anpresseinrichtung kann ferner eine bewegliche Manipulationseinrichtung aufweisen, insbesondere eine Halteeinrichtung für ein Werkzeug, z.B. einen Roboterarm, mittels der ein als Werkzeug dienender Anpresskopf an das Körperteil angelegt wird und den gewünschten Anpressdruck ausübt. Die Auflagefläche des Anpresskopfs kann insbesondere zwischen einem und mehreren Quadratzentimeter betragen, so dass insbesondere ein relativ lokalisierter Druck auf das Körperteil aufbringbar ist. Dadurch kann insbesondere nur ein einzelnes Blutgefäß in dem gewünschten Maße gestaut werden, oder eine kleinere Zahl von Blutgefäßen, was eine schonende Behandlung ermöglicht. Die Auflagefläche kann zudem einen Kraftsensor aufweisen, um den Anpressdruck auch auf diesem Wege kontrollieren zu können - zusätzlich zu der Messung mittels Gefäßstrukturmesseinrichtung. Die Auflagefläche kann insbesondere zwischen 1 cm² und 100 cm² betragen, insbesondere zwischen 1 cm² und 50 cm², vorzugsweisen zwischen 1 cm² und 10 cm².

Die Anpresseinrichtung kann einen Kraftsensor aufweisen, um den Anpressdruck zu messen. Der Wert des Anpressdrucks, insbesondere jener, der vorliegt, wenn das zu erkennende Blutgefäß des Patienten den gewünschten -die Lage und/oder Abmessungen charakterisierenden- Wert erreicht hat, kann in einer Datenspeichereinrichtung gespeichert werden. Die Datenspeichereinrichtung kann ein Teil der Erkennungsvorrichtung oder ein Teil der Behandlungsvorrichtung, insbesondere des Kanülierautomaten sein, der die Erkennungsvorrichtung aufweist.

Vorzugsweise ist die Anpresseinrichtung dazu eingerichtet, einen vorbestimmten, maximalen Wert des Anpressdrucks nicht zu übersteigen. Dieser Wert kann zuvor als solcher Wert ermittelt werden, bei dem der Blutfluss des gestauten Blutgefäßes bereits vollkommen geblockt oder bis zu einem bestimmten Wert gedrosselt wurde. Auf die Weise kann das Körperteil des Patienten schonend behandelt werden. Diese Sicherheitseinrichtung kann insbesondere bei Dialysepatienten oder anderen Erkrankungen vorteilhaft sein, bei denen ein Blutgefäß, insbesondere eine arteriovenöse Fistel, wiederholt punktiert werden muss und deshalb insbesondere schmerzempfindlich sein kann.

Vorzugsweise weist die Erkennungsvorrichtung eine Blutdruckmesseinrichtung auf. Die Anpresseinrichtung kann als Blutdruckmesseinrichtung eingerichtet sein.

Vorzugsweise wird der Anpressdruck auf einen Wert zwischen 40 mmHg und 100 mmHg eingestellt. Der Anpressdruck wird vorzugsweise so gewählt, dass der venöse Abstrom durch das zu erkennende Blutgefäß unterbrochen wird, nicht jedoch der arterielle Zustrom. Deshalb ist bevorzugt, dass der Anpressdruck kleiner ist als der diastolische Druck.

Die Anpresseinrichtung kann dazu ausgebildet sein, den Anpressdruck wiederholt aufzubringen, insbesondere mittels eines Kontaktabschnitts der Anpresseinrichtung, der wiederholt gegen das Körperteil gepresst wird. Durch ein Beklopfen des Körperteils wird in der Haut Histamin freigesetzt, was zur Rötung der Haut führt, wodurch sich das / die Blutgefäße erweitern. Das Blutgefäß wird auch durch diese Manipulation besser zur Darstellung gebracht.

Die Gefäßmanipulationseinrichtung kann als Wärmeübertragungseinrichtung ausgebildet sein, um Wärme auf das Körperteil des Patienten zu übertragen. Durch Wärme kann die Hautdurchblutung verbessert werden. Durch Wärme kann ein Gefäß geweitet werden, so dass eine nachfolgende Behandlung, insbesondere eine automatische Kanülierung verbessert werden kann. Die Gefäßmanipulationseinrichtung kann insbesondere einen Wärmeübertragungsabschnitt aufweisen, der dazu vorgesehen ist, mit der Haut in Kontakt zu stehen und die Wärme durch diffusiven Wärmetransport zu übertragen. Wie bereits beschrieben, kann eine Kisseneinrichtung als Wärmeübertragungseinrichtung ausgebildet sein, indem das in der Kisseneinrichtung enthaltene fluide Medium auf eine Solltemperatur erwärmt oder gekühlt wird. Die Kisseneinrichtung kann insbesondere als Hülle ausgebildet sein, insbesondere als Manschette, so dass die Hülle das Körperteil zumindest teilweise oder vollständig in zumindest einer Ebene umgibt, insbesondere röhrenartig umgibt. Dadurch kann ein besonders großflächiger thermischer Kontakt mit der Haut erzielt werden, um das Gewebe großflächig zur Durchblutung anzuregen. Optional kann die Hülle aufgepumpt bzw. mit Fluid angereichert werden, um einen Anpressdruck auszuüben, der der Blutstauung dienen kann und der andererseits den thermischen Kontakt zwischen Hülle und Körperteil verbessern kann. Die Hülle kann transparent sein und/oder kann eine Aussparung oder Öffnung aufweisen, um die Messung mittels der Gefäßstrukturmesseinrichtung zu ermöglichen. Die Wärmeübertragungseinrichtung kann insbesondere mindestens eine Temperiereinrichtung, und vorzugsweise mindestens einen Temperatursensor aufweisen, und insbesondere zur geregelten Einstellung der Temperatur ausgebildet sein.

Vorzugsweise weist die Wärmeübertragungseinrichtung einen Wärmestrahler auf, um Wärme durch Strahlung auf das Körperteil zu übertragen. Der Wärmestrahler kann ein Infrarotwärmestrahler sein. Der Wärmestrahler kann dazu eingerichtet sein, die Wärme gerichtet auf ein Körperteil zu strahlen, insbesondere auf einen Abschnitt des Körperteils, um die Wärme gezielt aufzubringen. Der Wärmestrahler kann beweglich im Behandlungsraum montiert sein.

Die Gefäßmanipulationseinrichtung kann eine bewegliche Stützeinrichtung zum beweglichen Stützen des Arms aufweisen. Dazu weist die Gefäßmanipulationseinrichtung vorzugsweise eine Aktuatoreinrichtung auf, insbesondere einen elektrischen Motor, um die bewegliche Stützeinrichtung zu bewegen. Diese Bewegung kann relativ zur Basis der Erkennungsvorrichtung erfolgen und kann eine rotatorische und/oder translatorische Bewegung sein. Durch die Bewegung des Körperteils lässt sich auch die Lage des Blutgefäßes in der gewünschten Weise verändern. Die Bewegung ist dabei der physiologischen Situation angepasst - das Körperteil darf nicht unphysiologisch bewegt werden. Nützlich kann eine solche Manipulation sein, um zum Beispiel bei einer Punktion des Blutgefäßes die Bewegung der punktierenden Kanüle komplementär zu ergänzen. Es ist aber auch bevorzugt, dass die Stützeinrichtung nicht beweglich ist.

Die Steuerungseinrichtung ist dazu eingerichtet, die Gefäßmanipulationseinrichtung in Abhängigkeit von den mittels der Gefäßstrukturmesseinrichtung gewonnenen Gefäßstrukturdaten zu steuern. Die Messung kann in einer höheren Frequenz erfolgen als die Manipulation. Vorzugsweise ist die Steuerungseinrichtung dazu eingerichtet, mittels der Gefäßstrukturmesseinrichtung eine -insbesondere genau eine- Messung von Gefäßstrukturdaten durchzuführen, und danach eine -insbesondere genau eine-Manipulation durchzuführen. Es kann zum Beispiel ausreichen und vorgesehen sein, dass nach Ermitteln der Gefäßdicke genau ein Anpressdruck mittels Anpresseinrichtung am Körperteil aufgebracht wird, der einem Erfahrungswert entspricht, mit dem ein solches Blutgefäß in typischer Weise gestaut und so erweitert wird, so dass es insbesondere für eine Punktion geeignet ist. Der Anpressdruck kann patientenindividuell sein, und kann zudem individuell für das Blutgefäß des Patienten gewählt sein, indem er z.B. aus historischen Patientendaten entnommen wurde, die insbesondere auch Gefäßstrukturdaten enthalten können.

Vorzugsweise ist die Steuerungseinrichtung dazu eingerichtet, die Gefäßmanipulationseinrichtung in Abhängigkeit von den Gefäßstrukturdaten zu regeln. Bei der Regelung wird eine bestimmte Lage und/oder Abmessung des Blutgefäßes als vorgegebene Sollgröße eines Regelkreises festgelegt. Die Lage und/oder Abmessung des Blutgefäßes werden als Messgröße des Regelkreises erfasst. Als Stellgröße des Regelkreises dient ein Parameter, mittels dem die Gefäßmanipulationseinrichtung gesteuert wird. Der Parameter kann ein den Anpressdruck charakterisierender Parameter sein, mittels dem bei einer als Anpresseinrichtung ausgebildeten Gefäßmanipulationseinrichtung das Maß von deren Aktivität bestimmt wird. Die bei der Regelung vorgesehenen wiederholten Messungen und Manipulationen erlauben eine besonders präzise Einstellung von Lage und/oder Abmessungen des Blutgefäßes.

Vorzugsweise ist die Erkennungsvorrichtung dazu ausgebildet, nach Durchführung der Manipulation den diese Gefäßmanipulation kennzeichnenden Wert, insbesondere den Wert des Anpressdrucks, zu speichern. Dieser Wert ist insbesondere jener, der vorliegt, wenn das zu erkennende Blutgefäß des Patienten den gewünschten -die Lage und/oder Abmessungen charakterisierenden- Wert erreicht hat. Er kann in einer Datenspeichereinrichtung gespeichert werden und kann insbesondere als Teil von Patientendaten gespeichert werden.

Vorzugsweise ist die Steuerungseinrichtung dazu eingerichtet, die Änderung oder die Regelung der Lage und/oder Abmessungen des Blutgefäßes innerhalb einer vorbestimmten Zeitspanne durchzuführen, insbesondere einer Zeitspanne von weniger als 60 Sekunden, vorzugsweise weniger als 30 Sekunden, vorzugsweise weniger als 20 Sekunden, vorzugsweise weniger als 10 Sekunden durchzuführen. Wird das Blutgefäß durch die Manipulation gestaucht, so wird durch eine limitierte, insbesondere eine kurze Manipulationszeit eine unphysiologische Belastung des Körperteils vermieden. Vorzugsweise wird auch eine nachfolgende Kanülierung -bei Anwendung der Erkennungsvorrichtung in einem Kanülierautomaten- innerhalb der genannten Zeitspanne durchgeführt, um die Manipulation und damit die physische Belastung des Körperteils so kurz wie möglich zu halten. Es ist bekannt, dass bestimmte Blutwerte bei einer längeren Stauung verändert werden können, was insbesondere bei einer nachfolgenden Diagnostik am Blut unerwünscht sein kann.

Vorzugsweise ist die Erkennungsvorrichtung gemäß der Erfindung oder eine ihrer hier beschriebenen Ausgestaltungen ein Bestandteil einer Behandlungsvorrichtung, insbesondere eines Kanülierautomaten. Der Kanülierautomat, bzw. die Steuerungseinrichtung des Kanülierautomaten, ist vorzugsweise dazu ausgebildet, die Kanülierung des mittels der Erkennungsvorrichtung erkannten und veränderten Blutgefäßes automatisch durchzuführen, nachdem die Lage und/oder die Abmessungen des Blutgefäßes in der gewünschten Weise durchgeführt wurde. Eine Behandlungsvorrichtung kann ferner eine nicht-invasive Behandlung des Blutgefäßes vornehmen.

Die Erkennungsvorrichtung kann dazu eingerichtet sein, vor, während und/oder nach der Manipulation des Blutgefäßes durch die Gefäßmanipulationseinrichtung mindestens ein Bild der Gefäßstruktur aufzunehmen, zum Beispiel mittels einer in der Gefäßstrukturmesseinrichtung vorgesehenen Bilderfassungseinrichtung, und dieses mindestens eine Bild als Gefäßstrukturdaten in einer Datenspeichereinrichtung zu speichern. In diesem Fall dient die Manipulation dazu, die visuelle Darstellung der Gefäßstruktur zu verbessern. Derartige Gefäßstrukturdaten können zum Nachweis der gesundheitlichen Entwicklung einer Gefäßstruktur, insbesondere einer Fistel, eingesetzt werden.

Die Erkennungsvorrichtung bzw. die Steuerungseinrichtung der Erkennungsvorrichtung, kann dazu eingerichtet sein, auf -insbesondere einer Patientendatenbank- gespeicherten Patientendaten zuzugreifen, um Informationen über vergangene Manipulationsdaten, insbesondere Stellgrößen der Gefäßmanipulationseinrichtung, insbesondere einen Anpressdruck, zu ermitteln. Ein Kanülierautomat kann dazu eingerichtet sein, aus solchen Patientendaten geeignete Kanülierungsprozessschritte bei der Kanülierung des Blutgefäßes des Patienten (historische Daten) zu ermitteln, und vorzugsweise die vorzunehmende Kanülierung, insbesondere die bei der programmgesteuerten automatischen Kanülierung verwendeten Programmparameter, in Abhängigkeit von diesen historischen Daten zu bestimmen. Solche historischen Daten enthalten insbesondere die Lage eines oder mehrerer Blutgefäße des Patienten, die zuvor mit der Erkennungsvorrichtung zur Messung der Lage und/oder Abmessungen mindestens des Blutgefäßes unter der Haut des Patienten (Gefäßstrukturmesseinrichtung) gemessen wurden, und die insbesondere als Patientendaten zur Verfügung stehen. Solche historischen Daten enthalten insbesondere Informationen über Lage und Beschaffenheit früherer Punktionsstellen am Köperteil des Patienten, die insbesondere als Patientendaten zur Verfügung stehen.

Die Erkennungsvorrichtung bzw. die Steuerungseinrichtung der Erkennungsvorrichtung, kann dazu eingerichtet sein, eine Identifizierung des zur Behandlung, insbesondere zur Punktion geeigneten Blutgefäßes unter der Haut des Körperteils des Patienten vorzunehmen, und insbesondere eine geeignete Einstichposition auf der Haut zur Punktion dieses Blutgefäßes zu wählen. Die Identifizierung kann zum Beispiel in einer Steuerungseinrichtung durch programmgesteuerte Analyse eines Bildes erfolgen, welches durch die Gefäßstrukturmesseinrichtung gewonnen wurde.

Im Sinne der Erfindung ist eine "Kanüle" ein röhrenförmiger Körper, insbesondere eine starre oder flexible Injektionsnadel, mit einem Lumen, mit einer Geometrie und mit Außenabmessungen, die geeignet sind, um bei einer Kanülierung eines Blutgefäßes verwendet zu werden. Vorzugsweise weist die Kanüle eine Hohlnadel und ein Anschlussteil auf.

Die bereits vorausgehend genannten möglichen Vorteile sowie Ausführungsformen, Weiterbildungen oder Varianten der Erfindung gelten entsprechend auch für den erfindungsgemäßen Kanülierautomaten.

Ein Kanülierautomat ist eine Vorrichtung, die mindestens einen Prozessschritt der Kanülierung eines Blutgefäßes eines Patienten, oder einige oder alle dafür vorgesehenen Prozessschritte automatisch, das heißt zumindest zeitweise oder ununterbrochen, ohne Eingriff eines menschlichen Bedieners, z.B. medizinischen Personals, durchführt. Dies erfolgt insbesondere dadurch, dass die Programmparameter der automatischen Kanülierung vom System und/oder vom Benutzer entsprechend gewählt werden. Ein Prozessschritt der Kanülierung wird insbesondere durch einen für diesen Prozessschritt spezifisch eingerichteten Apparatebestandteil des Kanülierautomaten, zum Beispiel eine Werkzeugeinrichtung, technisch implementiert und ist ausgewählt aus der Gruppe der möglichen Prozessschritte P1, P2, P3 ... umfassend, ohne Festlegung einer Reihenfolge durch diese Nummerierung:
P1: Verwendung eines vor Beginn der automatisierten Kanülierung in Abhängigkeit von der angemeldeten Patientenkennung gewählten Zubehörsets für die Durchführung der Kanülierung; diese Auswahl kann zuvor erfolgt sein mittels eines optionalen Bestückungssystems des Systems zur Auswahl eines Zubehörsets und/oder Bestückung eines Zubehörträgers, insbesondere Zubehörbehälters; das Zubehörset kann zuvor in Abhängigkeit von der angemeldeten Patientenkennung von einer optionalen Aussonderungsvorrichtung des Systems durch Aussonderung des im Zubehörset enthaltenen Zubehörs aus einer optionalen Lagervorrichtung des Systems zur Lagerung von Zubehör bereitgestellt worden sein; das Zubehörset kann ein oder mehrere medizinische Zubehöre, insbesondere Mullbinden, Tupfer, Klebeband, enthalten; die Zubehörteile dieses Zubehörsets können in Abhängigkeit von der angemeldeten Patientenkennung und/oder in Abhängigkeit von patientenspezifischen Behandlungsdaten, die aus der angemeldeten Patientenkennung abgeleitet sein können, gewonnen worden sein; die Verwendung dieses Zubehörsets durch den Kanülierautomaten ist ein Arbeitsschritt der automatisierten Kanülierung und kann vorsehen, dass das Zubehör des Zubehörsets aus vorbestimmten Positionen eines Zubehörträgers /-behälters automatisch entnommen wird, insbesondere indem die entsprechenden Programmparameter in Abhängigkeit von der angemeldeten Patientenkennung und geeignet zur Entnahme gewählt werden; dazu wird insbesondere eine optionale Bestückungseinrichtung des Kanülierautomaten verwendet, die zur Entnahme des Zubehörs aus dem Zubehörbehälter eingerichtet ist und/oder zur Bestückung eines oder mehrere optionaler Werkzeugeinrichtungen des Kanülierautomaten eingerichtet ist;
P2: räumliche Fixierung eines Körperteils des Patienten, welches das Blutgefäß, insbesondere eine arteriovenöse Fistel, aufweist; die Programmparameter der automatisierten Kanülierung können hierbei in Abhängigkeit von der angemeldeten Patientenkennung, also patientenindividuell, gewählt sein, indem diese Programmparameter die Lage oder die Abstände von einer oder mehreren optionalen Fixiereinrichtungen des Kanülierautomaten in Abhängigkeit von einer zuvor bestimmten Lage oder von zuvor am Körperteil dieses Patienten bestimmten Abständen so einstellen, dass eine geeignete Fixierung erreicht wird; die Fixierung erfolgt im Behandlungsraum des Kanülierautomaten, in dem das Körperteil des Patienten für die nachfolgende mindestens eine Punktion gelagert wird;
P3: Verwendung von -insbesondere einer Patientendatenbank- gespeicherten Patientendaten, um Informationen über vergangene Kanülierungsprozessschritte am Blutgefäß des Patienten (historische Daten) zu ermitteln, und vorzugsweise die vorzunehmende Kanülierung, insbesondere die dabei verwendeten Programmparameter, in Abhängigkeit von diesen historischen Daten zu bestimmen; solche historischen Daten enthalten insbesondere die Lage eines oder mehrerer Blutgefäße des Patienten, die zuvor mit einer optionalen Messeinrichtung des Kanülierautomaten zur Messung der Lage und/oder Abmessungen mindestens eines Blutgefäßes unter der Haut des Patienten (Gefäßstrukturmesseinrichtung) gemessen wurden, und die insbesondere als Patientendaten zur Verfügung stehen; solche historischen Daten enthalten insbesondere Informationen über Lage und Beschaffenheit früherer Punktionsstellen am Köperteil des Patienten, die insbesondere als Patientendaten zur Verfügung stehen; die Gefäßstrukturmesseinrichtung kann zur Erfassung der Lage und/oder Abmessungen mindestens eines Blutgefäßes unter der Haut des Patienten mittels Ultraschall oder mittels optischer Strahlung ausgebildet sein;
P4: Identifizierung des zur Blutentnahme geeigneten Blutgefäßes unter der Haut des Körperteils des Patienten, insbesondere Auswählen einer geeigneten Einstichposition auf der Haut zur Punktion dieses Blutgefäßes; die Programmparameter der automatisierten Kanülierung können hierbei in Abhängigkeit von der angemeldeten Patientenkennung, also patientenindividuell, gewählt sein, indem die für den angemeldeten Patienten vorgesehene Kanülierung anhand von mindestens einem Behandlungsparameter gewählt wurde, der patientenspezifisch ist; zum Beispiel kann ein Patient für eine Hämodialyse vorgesehen sein; ein Behandlungsparameter kann den Bedarf für eine Hämodialyse bei diesem Patienten codieren; durch Auswertung des Behandlungsparameters kann die Kanülierung eines arteriovenösen Blutgefäßes geplant werden; dieses ist zu identifizieren; die Identifizierung kann zum Beispiel im Steuerungssystem durch programmgesteuerte Analyse eines Bildes erfolgen, welches durch eine Gefäßstrukturmesseinrichtung gewonnen wurde;
P5: Desinfektion der Haut des Körperteils des Patienten, welches das Blutgefäß aufweist; die Programmparameter der automatisierten Kanülierung können hierbei in Abhängigkeit von der angemeldeten Patientenkennung, also patientenindividuell, gewählt sein, indem eine für den Hauttyp oder die Hautmorphologie des Patienten spezifisch ausgewähltes Desinfektionsverfahren ausgewählt wird, das zum Beispiel durch die Dauer der Behandlung, oder die Menge und Zusammensetzung des verwendeten Desinfektionsverfahrens charakterisiert ist; auch Behandlungsdaten können berücksichtigt werden, die für den Patienten spezifisch sind; es kann für die genannte Desinfektion eine optional beim Kanülierautomaten oder separat davon vorgesehene Desinfektionseinrichtung eingesetzt werden, welche zur Ausführung der genannten Funktion eingerichtet ist; Hauttyp oder die Hautmorphologie des Patienten sind insbesondere als Patientendaten der Patientendatenbank dem System vorzugsweise bekannt;
P6: Physische Behandlung zur Vorbereitung der Punktion des Körperteils des Patienten, welches das Blutgefäß aufweist, insbesondere Stauen des Blutflusses des Körperteils, Druckausübung auf das Körperteil, Temperierung des Körperteils, Positionieren des fixierten Körperteils; die Programmparameter der automatisierten Kanülierung können hierbei in Abhängigkeit von der angemeldeten Patientenkennung, also patientenindividuell, gewählt sein, indem Vorbereitungsdaten herangezogen werden, die für die geplante Behandlung des Patienten, z.B. eine Hämodialyse, spezifisch sind, oder die als vorbekannte Vorbereitungsdaten der Patientendatenbank entnommen werden können; diese Vorbereitung der Punktion des Körperteils wird insbesondere von einer optional vorgesehenen Vorbereitungseinrichtung des Kanülierautomaten durchgeführt, der für diesen Zweck entsprechend eingerichtet ist;
P7: besonders bevorzugt: Punktion des Blutgefäßes, insbesondere einer arteriovenösen Fistel; vorzugsweise werden für eine Blutentnahme aus dem Blutgefäß eine erste Punktion und Kanülierung und für die Blutrückführung in das Blutgefäß eine zweite Punktion und Kanülierung automatisch durchgeführt, insbesondere bei der Hämodialyse; die Programmparameter der automatisierten Kanülierung können hierbei in Abhängigkeit von der angemeldeten Patientenkennung, also patientenindividuell, gewählt sein, indem durch die Programmparameter eine patientenabhängige Bewegungssteuerung für einen optional beim Kanülierautomaten vorgesehenen robotergesteuerten Werkzeugarm definiert wird, mittels der zum Beispiel ein medizinisches Zubehör wie etwa eine Injektionsnadel vom Werkzeugarm gegriffen und am Körperteil positioniert wird, wobei die Injektionsnadel zuvor patientenspezifisch ausgewählt und vorbereitet wurde; zwei Kanülierautomaten können für die Punktion von Blutgefäßen an unterschiedlichen Körperteilen eingerichtet sein, indem z.B. ein erster Kanülierautomat für die Kanülierung an einem Arm eingerichtet sein kann und ein zweiter Kanülierautomat für die Kanülierung an einem Bein eingerichtet sein kann; die Auswahl des geeigneten Kanülierautomaten kann patientenspezifisch und/oder behandlungsspezifisch erfolgen; möglich ist z.B. auch, dass an beiden Armen (Beinen) jeweils ein Kanulierautomat eingesetzt werden kann.
P8: Blutentnahme aus dem punktierten Blutgefäß und Überführen des Blutes in mindestens eine Bluttransporteinrichtung oder in mindestens ein Probenbehältnis; die Programmparameter der automatisierten Kanülierung können hierbei in Abhängigkeit von der angemeldeten Patientenkennung, also patientenindividuell, gewählt sein, indem in Abhängigkeit von patientenspezifischen Behandlungsdaten eine geeignete Bluttransporteinrichtung oder ein geeignetes Probenbehältnis vorausgewählt wurde und nun in geeigneter Weise vom Kanülierautomaten verwendet wird; der Kanülierautomat und das Steuerungssystem können mittels entsprechender Wahl der Programmparameter dazu eingerichtet sein, mindestens ein Probenbehältnis in Abhängigkeit von Behandlungsdaten für die nachfolgende, vorzugsweise automatische und vom System gesteuerte Bearbeitung, insbesondere Diagnostik, bereitzustellen;
P9: Greifen einer Kanüle mittels einer Greifervorrichtung des Kanülierautomaten.

Unter dem Begriff "Kanülierung" wird ein Vorgang verstanden, bei dem eine Kanüle durch Punktion der Haut und Punktion der Wand eines Blutgefäßes im Körperteil des Patienten in das Blutgefäß eingeführt wird, so dass das distale Ende der Kanüle im Blutgefäß angeordnet ist, und das proximale Ende der Kanüle außerhalb des Körperteils angeordnet ist, so dass eine Fluidverbindung zwischen Kanüle und Blutgefäß geschaffen ist, über die Fluid, insbesondere Blut und/oder fluide Medien, über die Fluidverbindung ausgetauscht werden können. Der "Austausch" des Fluids bedeutet in diesem Kontext, dass das Fluid aus dem Blutkreislauf des Patienten in ein extrakorporales, also außerhalb des Patientenkörpers gelegenes Fluidsystem, insbesondere zur Fluidlagerung oder Fluidführung, überführt wird, und/oder beinhaltet, dass das Fluid aus dem extrakorporalen System in den Blutkreislauf überführt wird.

Patienten mit chronischer Erkrankung benötigen regelmäßig wiederholte Punktionen der Blutgefäße, um die erforderliche Behandlung sicherzustellen. Eine solche chronische Erkrankung ist das Nierenversagen, das unter anderem zum Ausfall der natürlichen Blutreinigungsfunktion führt. Diese kann durch technische Lösungen ersetzt werden. Hämodialysegeräte sind als künstliche Nieren dienende extrakorporale Filteranlagen, in die das Blut des Patienten geleitet wird, um dort gereinigt und aufbereitet zu werden, bevor es in den Blutkreislauf des Patienten zurückgeführt wird. Die Entnahme des Blutes und die Rückführung des Blutes erfolgt in der Regel über eine auf chirurgischem Wege in einem Arm oder Bein des Patienten subkutan geschaffene, künstliche Verbindung zwischen einer Vene und einer Arterie. Diese Verbindung kann aus einem dazu vorbereiteten eigenen Gefäßabschnitt des Patienten oder aus einem künstlichen Material bestehen und wird als Fistel bzw. arteriovenöse Fistel (AV-Fistel, AVF) bezeichnet.

Der am meisten genutzte permanente Gefäßzugang bei chronischen Hämodialysepatienten ist eine native arteriovenöse Fistel. Nach dem Legen der nativen arteriovenösen Fistel wird diese durch den erhöhten Blutdurchfluss stärker, wodurch die wiederholte Punktion für die Dialysebehandlung einfacher wird.

Hämodialyse muss regelmäßig durchgeführt werden, typischer Weise im Abstand weniger Tage. Dies führt zu einer hohen mechanischen Belastung des Gefäßes, bzw. der arteriovenösen Fistel. Um den Zugang zu einem Gefäß bzw. einer arteriovenösen Fistel zu legen, sind verschiedene Techniken bekannt, die darauf abzielen, das Gefäß im Laufe der wiederkehrenden Punktionen möglichst zu schonen. Bei der Strickleiterpunktion wird für jede Behandlung eine neue Kanülierungsstelle gesucht, die von der vorhergehenden ein Stück weit entfernt liegt, z.B. ca. 2 cm. Üblicherweise wird bei diesem Verfahren die Punktionsserie am unteren Ende des Gefäßes begonnen und dann nach oben fortgesetzt, bis das obere Ende erreicht ist und der Vorgang wieder von unten gestartet wird. Hierbei muss vom Behandler das Positionierschema genau eingehalten werden, um die Ausheilung der punktierten Gefäßpositionen zu ermöglichen. Dagegen wird bei der Knopflochtechnik eine Nadel immer an genau derselben Stelle mit demselben Winkel eingeführt. Mit der Zeit entsteht so ein Narbenzylinder, in welchem der gebildete Thrombus bei Punktion immer wieder verdrängt wird und dadurch belastbarer wird. Es wurde beobachtet, dass sich Ergebnisse der Knopflochtechnik verbessern lassen, wenn die Punktion stets von demselben Behandlungspersonal durchgeführt wird. Aus diesem Grund ist die Verwendung eines Kanülierautomaten besonders vorteilhaft.

Durch die Häufigkeit der Punktionen bei Hämodialysepatienten ist die arteriovenöse Fistel unabhängig von der Punktionstechnik generell einer besonders hohen Belastung ausgesetzt, die zu Veränderungen der Hautoberfläche und der Beschaffenheit der arteriovenösen Fistel und ihres Verlaufs führen kann. Die vorliegende Erfindung erlaubt ein dosiertes Optimieren der Lage und/oder Abmessungen des Blutgefäßes, so dass insbesondere eine automatische Kanülierung schonend, schnell und effizient durchführbar ist.

Ein Vorteil des Kanülierautomaten mit Erkennungsvorrichtung kann zudem insbesondere darin liegen, dass dieser, insbesondere bei Behandlung von chronischen Erkrankungen - insbesondere bei Hämodialysepatienten -, durch die automatisierte Kanülierung das medizinische Personal entlasten kann und/oder eine gleich bleibend hohe Präzision bei der Kanülierung ermöglicht, wodurch insbesondere die Behandlungsqualität und/oder Behandlungssicherheit gesteigert werden kann.

Die bereits vorausgehend genannten möglichen Vorteile sowie Ausführungsformen, Weiterbildungen oder Varianten der vorhergehenden Aspekte der Erfindung gelten entsprechend auch für das erfindungsgemäße Verfahren. Umgekehrt gelten mögliche Vorteile sowie Ausführungsformen, Weiterbildungen oder Varianten des Verfahrens entsprechend auch für die vorhergehenden Aspekte der Erfindung.

Unter "eingerichtet" ist im Sinne der Erfindung zu verstehen, dass eine Vorrichtung nicht nur prinzipiell geeignet ist eine bestimmte Funktion zu erfüllen - etwa erst nachdem ein bestimmter Programmcode aufgespielt worden ist, also die Vorrichtung programmiert worden ist, oder die Vorrichtung in bestimmter Weise ausgeformt worden ist -, sondern die Vorrichtung bereits alle Mittel besitzt, um die Funktion tatsächlich zu erfüllen. Vorzugsweise ist dazu die Vorrichtung bereits mit einem Programmcode für diese Funktion programmiert und/oder bereits so geformt und/oder so angeordnet und/oder weist dazu bereits eine solche Konfiguration auf, dass die Vorrichtung die Funktion tatsächlich erfüllt.

Unter einer "Behandlung eines Patienten" ist im Sinne der Erfindung zumindest ein medizinisches, d.h. insbesondere therapeutisches oder diagnostisches, oder kosmetisches Verfahren zu verstehen, welches den Körper und/oder die Gesundheit des Patienten verändert oder mittels dessen der Gesundheitszustand des Patienten bestimmt wird. Eine Behandlung ist insbesondere eine Verabreichung von Arzneimitteln, eine Kanülierung, ein Blutreinigungsverfahren wie die Dialyse, eine Operation und/oder eine Untersuchung des Patienten.

Eine "Gruppe von Behandlungen" im Sinne der Erfindung können jeweils bestimmte Operationen, die Therapie einer bestimmten Erkrankung, die Eingangsuntersuchung eines Patienten oder eine Dialyse-Behandlung, welche wiederum Untergruppen, insbesondere Behandlung mittels Hämodialyse, Hämofiltration, Hämodiafiltration, Hämoperfusion oder Peritonealdialyse, aufweisen kann, sein. Eine weitere mögliche Behandlungsgruppe ist die Apherese.

Unter einer "an der Behandlung beteiligten Person" ist im Sinne der Erfindung insbesondere eine behandelnde Person, etwa ein Arzt oder eine Ärztin, oder eine Person, die die Behandlung unterstützt, etwa eine Pflegekraft, zu verstehen. Insbesondere kann auch der zu behandelnde Patient selbst eine an der Behandlung beteiligte Person oder behandelnde Person sein.

Eine datenverarbeitende Steuerungseinrichtung der Erkennungsvorrichtung und/oder des Kanülierautomaten weist eine Datenverarbeitungsvorrichtung auf.

Unter einer "Datenverarbeitungsvorrichtung" ist eine Vorrichtung zu verstehen, welche eingerichtet ist, Daten zu verarbeiten, d.h. insbesondere Daten zu empfangen, empfangene Daten zu speichern, gespeicherte Daten auszulesen, empfangene und/oder gespeicherte bzw. ausgelesene Daten mittels logischer und/oder mathematischer Operationen zu transformieren, transformierte Daten zu speichern und/oder transformierte bzw. ausgelesene Daten auszugeben. Vorzugsweise ist eine solche Datenverarbeitungsvorrichtung programmierbar, d.h. insbesondere dass die Verfahren zum Verarbeiten der Daten zumindest teilweise durch einen Programmcode bestimmt werden und dieser Programmcode zumindest teilweise veränderbar ist.

Vorzugsweise ist die Datenverarbeitungsvorrichtung ein handelsüblicher Mikroprozessor oder Computer. Weiter bevorzugt weist die Datenverarbeitungsvorrichtung wenigstens einen Datenprozessor - also eine Recheneinheit -, einen nicht-flüchtigen - d.h. insbesondere dauerhaften - Datenspeicher, insbesondere eine Festplatte, einen Read-Only-Memory (ROM) oder ein Laufwerk mit Datenträger, sowie wenigstens eine Hardwareschnittstelle auf. Auch vorzugsweise weist die Datenverarbeitungsvorrichtung einen flüchtigen elektrischen Datenspeicher, insbesondere als Arbeitsspeicher, auf, vorzugsweise einen Halbleiterspeicher, insbesondere mit integrierten Kondensatoren und/oder Flip-Flops (bistabile Multivibratoren) zur Datenspeicherung, etwa Dynamisches RAM oder statisches RAM.

Im Sinne der Erfindung ist eine "Datenspeichervorrichtung" oder "Datenspeichereinrichtung" eine Vorrichtung zum Speichern von Daten. Diese ist insbesondere zur Ausbildung einer Datenverbindung mit einer weiteren Vorrichtung, insbesondere einer Datenverarbeitungsvorrichtung, eingerichtet und/oder weist eine Datenverbindung mit der weiteren Vorrichtung auf, wobei mittels der Datenverbindung Daten von der weiteren Vorrichtung zur Datenspeichervorrichtung zum Speichern übertragen werden können und/oder zum Abrufen von gespeicherten Daten diese von der Datenspeichervorrichtung zur weiteren Vorrichtung übertragen werden können. Vorzugsweise weist die Datenspeichervorrichtung wenigstens einen nicht-flüchtigen Datenspeicher auf. Auch vorzugsweise weist die Datenspeichervorrichtung wenigstens einen flüchtigen elektrischen Datenspeicher auf.

Eine Datenverbindung verbindet insbesondere zwei datenverarbeitende Einheiten, insbesondere zwei Datenverarbeitungseinrichtungen oder -vorrichtungen, in der Weise, dass Daten zwischen den Einheiten ausgetauscht werden können, entweder unidirektional oder bidirektional. Die Datenverbindung kann leitungsgebunden realisiert sein oder drahtlos, insbesondere als Funkverbindung. Eine Datenfernverbindung verbindet insbesondere zwei datenverarbeitende Einheiten, insbesondere zwei Datenverarbeitungseinrichtungen, die entfernt voneinander angeordnet sind, die also insbesondere nicht Bestandteil desselben Geräts, insbesondere derselben Benutzerschnittstelleneinrichtung oder desselben Steuerungssystems sind, falls die genannten Geräte als separate Geräte ausgeführt sind. Eine Datenverbindung, insbesondere eine Datenfernverbindung, eines Geräts mit einem anderen Gerät wird vorzugsweise über eine direkte Verbindung der beiden Geräte realisiert, oder eine mittelbare Verbindung der beiden Geräte, so dass ein drittes Gerät zwischen die beiden Geräte geschaltet ist, um die Daten weiterzuvermitteln. Eine Datenfernverbindung kann insbesondere über ein Netzwerk aus Computern realisiert sein, bei denen die über die Datenfernverbindung verbundenen Geräte über das Netzwerk verbunden sind. Das Netzwerk kann ein beschränktes Netzwerk sein, z.B. ein Intranet, oder kann ein weltweites Netzwerk sein, insbesondere ein WAN und/oder das Internet.

Im Sinne der Erfindung dient eine "Schnittstelleneinrichtung" der Verbindung von zwei Einheiten - insbesondere auch von Systemen, Vorrichtungen, Einrichtungen oder Mechanismen, insbesondere mit solchen Einheiten -, die jeweils Signale, insbesondere Informationen, insbesondere Daten, verarbeiten, also insbesondere senden und/oder empfangen, können. Eine Schnittstelleneinrichtung kann wenigstens eine Hardwareschnittstelle aufweisen und insbesondere als Bestandteil in einer physischen Geräteeinheit integriert sein.

Der Begriff "Behandlung einer Laborprobe" bedeutet insbesondere, dass eine Laborprobe, insbesondere eine Blutprobe oder ein Blutvolumen, bewegt, und/oder transportiert und/oder untersucht und/oder verändert wird, insbesondere in ihrer Zusammensetzung, physikalisch, chemisch, biochemisch oder auf andere Weise verändert wird.

Die Erfindung bezieht sich ferner auf ein Verfahren zur automatischen Erkennung und Manipulation eines Blutgefäßes unter der Haut eines Körperteils eines Patienten, insbesondere ein Verfahren zum Betreiben einer Erkennungsvorrichtung, insbesondere einer erfindungsgemäßen Erkennungsvorrichtung, aufweisend die Schritte: - Erkennung der Lage und/oder Abmessungen eines Blutgefäßes in einem Behandlungsraum durch Messung von Gefäßstrukturdaten des Blutgefäßes im Behandlungsraum; - optional: Vergleich der Gefäßstrukturdaten mit Vergleichsdaten, die insbesondere vorbestimmte Referenzwerte der Lage und/oder Abmessung des Blutgefäßes enthalten; optional: - Anwenden eines Kriteriums, um aus diesem Vergleich ein Ergebnis zu ermitteln; - Änderung von Lage und/oder Abmessung des Blutgefäßes mittels einer Gefäßmanipulationseinrichtung, gesteuert durch eine Steuerungseinrichtung in Abhängigkeit von den Gefäßstrukturdaten.

Vorzugsweise weist die Erkennungsvorrichtung, insbesondere eine die Erkennungsvorrichtung aufweisende Behandlungsvorrichtung, mindestens eine der folgenden Komponenten auf: eine Benutzerschnittstelleneinrichtung, mit der ein Benutzer mindestens eine Dateneingabe vornehmen kann, die von der Steuerungseinrichtung oder deren Programmcode verarbeitet wird, und/oder mit der an den Benutzer eine Information ausgegeben werden kann, wobei die Benutzerschnittstelleneinrichtung ein Display, insbesondere Touchscreen, Lautsprecher und/oder ein Eingabegerät wie z.B. eine Tastatur aufweisen kann; ein Gehäuse, in das die Steuerungseinrichtung, der Behandlungsraum, die Gefäßstrukturmesseinrichtung, und/oder die Gefäßmanipulationseinrichtung eingebaut ist/sind, wobei das Gehäuse eine Öffnung oder eine Türeinrichtung aufweisen kann, über die der Behandlungsraum zugänglich ist, um das Körperteil des Benutzers aufzunehmen; eine Basis, insbesondere ein Halterahmen, der die Bestandteile der Erkennungsvorrichtung oder mindestens eines dieser Bestandteile trägt; eine Spannungsversorgung zur Versorgung der elektrischen Teile der Erkennungsvorrichtung mit Energie; eine Kommunikationseinrichtung zum Austausch von Daten mit einer externen Datenverarbeitungsvorrichtung, insbesondere über eine Datenfernverbindung.

Die Erfindung bezieht sich ferner auf ein datenverarbeitendes System, mit einer Erkennungsvorrichtung gemäß der vorliegenden Beschreibung, und/oder mit einer diese Erkennungsvorrichtung aufweisenden Behandlungsvorrichtung, insbesondere Kanülierautomaten, und mit mindestens einem externen Datenverarbeitungsvorrichtung, die mit der Erkennungsvorrichtung und/oder der Behandlungsvorrichtung zum Datenaustausch vernetzt sind, insbesondere über eine Datenverbindung oder eine Datenfernverbindung. Das System kann ferner eine Datenspeichervorrichtung als Bestandteil aufweisen, die zum Datenaustausch mit mindestens einem anderen Systembestandteil vernetzt ist. Die Datenspeichervorrichtung kann eine Patientendatenbank enthalten, in der Patientendaten gespeichert sind und abrufbar sind.

Die Erfindung bezieht sich ferner auf ein Verfahren zur automatischen Kanülierung eines Blutgefäßes unter der Haut eines Körperteils eines Patienten, insbesondere ein Verfahren zum Betreiben eines Kanülierungsautomaten, insbesondere eines erfindungsgemäßen Kanülierungsautomaten, aufweisend die Schritte des erfindungsgemäßen Verfahrens zur automatischen Erkennung und Manipulation eines Blutgefäßes unter der Haut eines Körperteils eines Patienten, und aufweisend den/die Schritte: - automatische Kanülierung des mittels der Gefäßmanipulationseinrichtung manipulierten Blutgefäßes; -optional: Durchführen dieser Kanülierung innerhalb einer vorbestimmten, limitierten Zeitspanne.

Weitere Vorteile, Merkmale und Anwendungsmöglichkeiten der vorliegenden Erfindung ergeben sich aus der nachfolgenden detaillierten Beschreibung wenigstens eines Ausführungsbeispiels und/oder aus den Figuren. Gleiche Bauteile der Ausführungsformen werden im Wesentlichen durch gleiche Bezugszeichen gekennzeichnet, falls dies nicht anders beschrieben wird oder sich nicht anders aus dem Kontext ergibt. Die Figuren zeigen folgende Ausführungsbeispiele der Erfindung:
Figuren 1 bis 5 zeigen jeweils eine Seitenansicht der erfindungsgemäßen Erkennungsvorrichtung gemäß einem bevorzugten Ausführungsbeispiel, bei dem jeweils die Gefäßmanipulationseinrichtung als Anpresseinrichtung ausgeführt ist.
Figuren 6 bis 8 zeigen jeweils eine Seitenansicht der erfindungsgemäßen Erkennungsvorrichtung gemäß einem bevorzugten Ausführungsbeispiel, bei dem jeweils die Gefäßmanipulationseinrichtung als Wärmeübertragungseinrichtung ausgeführt ist.
Figur 9 zeigt eine Seitenansicht der erfindungsgemäßen Erkennungsvorrichtung gemäß einem bevorzugten Ausführungsbeispiel, bei dem die Gefäßmanipulationseinrichtung als bewegliche Stützeinrichtung zum beweglichen Stützen des Arms ausgeführt ist.
Figur 10 zeigt eine Seitenansicht des erfindungsgemäßen Kanülierautomaten gemäß einem bevorzugten Ausführungsbeispiel, der mit einer erfindungsgemäßen Erkennungsvorrichtung ausgerüstet ist.
Figur 11 zeigt ein Ausführungsbeispiel des erfindungsgemäßen Verfahrens.
Figur 12 zeigt ein Beispiel für eine Änderung der Abmessung eines Blutgefäßes, bewirkt durch eine erfindungsgemäße Erkennungsvorrichtung.
Figur 13 zeigt ein Beispiel für eine Änderung der Lage eines Blutgefäßes, bewirkt durch eine erfindungsgemäße Erkennungsvorrichtung.

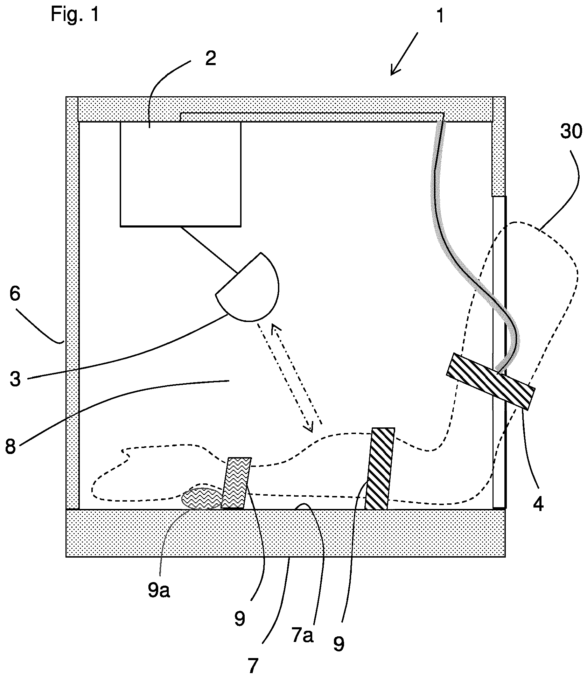

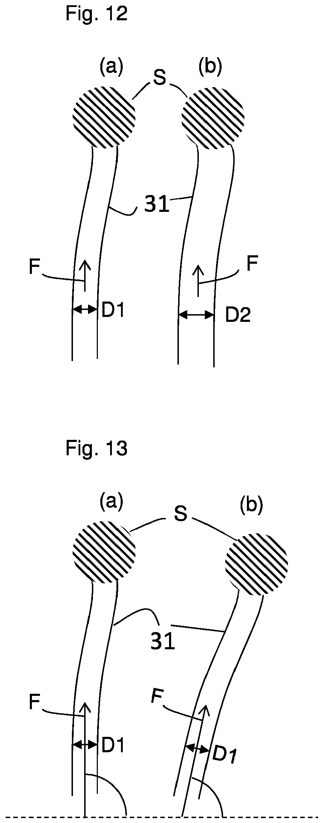

Figur 1 zeigt eine Erkennungsvorrichtung 1 zur Erkennung und Manipulation eines Blutgefäßes unter der Haut eines Körperteils eines Patienten. Der Arm 30 des Patienten ist mittels Fixierbändern 9 an einer mittels Körperteilführungspolstern 9a gepolsterten Körperteilauflage 7a, hier eine Armauflage, fixiert. Das Blutgefäß 31, siehe Figuren 12, 13, kann insbesondere eine arteriovenöse Fistel sein, die der Patient aufgrund einer Hämodialysebehandlung besitzt. Eine vollständige Blockade oder teilweise Stauung des Blutgefäßes an einer Position S, die bezüglich des zu erkennenden Abschnitts des Blutgefäßes stromaufwärts in Flußrichtung F gelegen ist, kann eine Erkennung und insbesondere nachfolgende Behandlung, insbesondere Kanülierung, des Abschnitts des Blutgefäßes verbessern.

Die Erkennungsvorrichtung weist eine Basisplattform 7 auf, mit einem offenen Rahmen 6 und einen Behandlungsraum 8 zur Aufnahme des Körperteils. Die Erkennungsvorrichtung 1 weist eine datenverarbeitende Steuerungseinrichtung 2 auf, eine Gefäßstrukturmesseinrichtung 3 zur Erkennung der Lage und/oder Abmessungen durch Messung von Gefäßstrukturdaten des Blutgefäßes im Behandlungsraum. Die Gefäßstrukturmesseinrichtung 3 ist hier eine Ultraschallmesseinrichtung.

Die Erkennungsvorrichtung 1 weist zudem eine als Anpresseinrichtung ausgebildete Gefäßmanipulationseinrichtung 4 zur Änderung der Abmessung des Blutgefäßes auf. Die Anpresseinrichtung 4 ist eine luftbefüllbare Arm-Hohlmanschette 4, und weist eine elektrisch betriebene Luftpumpe (nicht gezeigt) auf, um den Druck in der Arm-Hohlmanschette 4 zu erhöhen, bis eine bestimmte Lage und/oder Dicke des Blutgefäßes erreicht ist. Die Arm-Hohlmanschette 4 kann einen Kraft- bzw. Drucksensor (nicht gezeigt) aufweisen, um den mittels der Arm-Hohlmanschette auf das Körperteil, hier den Oberarm, aufgebrachten Druck zu messen und bei Überschreiten eines zulässigen Maximaldrucks die Gefäßmanipulation zu stoppen.

Die Steuerungseinrichtung 2 ist dazu eingerichtet, die Gefäßmanipulationseinrichtung 4 in Abhängigkeit von den mittels der Gefäßstrukturmesseinrichtung 3 gemessenen Gefäßstrukturdaten zu steuern. Die Steuerungseinrichtung ermittelt aus den Gefäßstrukturdaten, ob das Blutgefäß eine vorbestimmte Dicke D2 aufweist, die für eine nachfolgende Behandlung, insbesondere eine automatische Kanülierung dieses Blutgefäßes mit dieser Dicke geeignet ist. Die Steuerungseinrichtung 2 ist hier dazu eingerichtet, den Anpressdruck in dem Fall zu erhöhen, in dem das Kriterium erfüllt ist, dass die gemessene Dicke D1 des Blutgefäßes kleiner ist als D2. Die Druckerhöhung kann in inkrementellen Schritten unter ständiger Messung mittels der Gefäßstrukturmesseinrichtung 3 erfolgen. Alternativ oder zusätzlich kann die Druckerhöhung einen vorbestimmten, auf Erfahrungswerten beruhenden, größeren Schritt der Druckerhöhung beinhalten, der zum Beispiel vor einer inkrementellen Erhöhung oder Erniedrigung des Anpressdrucks aufgebracht wird, um den Erfolg der Blutstauungsmaßnahme zu beschleunigen.

Sobald in Folge der Erhöhung (und/oder Erniedrigung) des Anpressdrucks am Blutgefäß die Dicke D2 gemessen wird, wird die Änderung des Anpressdrucks gestoppt bzw. der Anpressdruck in der Weise geregelt, dass die gemessene Dicke konstant bei D2 bleibt, zumindest für eine vorbestimmte oder gesteuerte Zeitspanne. Innerhalb dieser Zeitspanne kann eine weitere Behandlung des Blutgefäßes erfolgen, insbesondere eine Kanülierung.

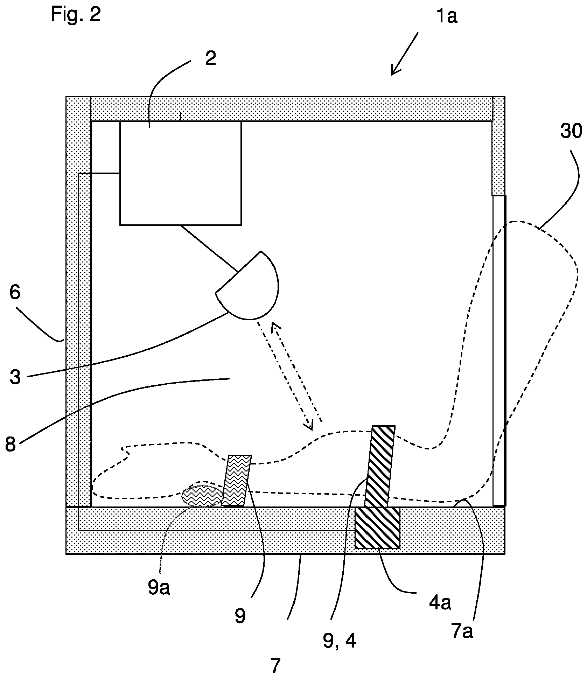

In Figur 2 ist die Erkennungsvorrichtung 1a gezeigt, deren Gefäßmanipulationseinrichtung 4 ebenfalls eine Anpresseinrichtung ist, insbesondere ein um den Arm 30 herumführendes und mittels Elektromotor 4a festzurrbares Abbindeband 4, das gleichzeitig als Fixierband zum Fixieren des Arms auf der Auflage 7a dient. Der Elektromotor 4a wird von der Steuerungseinrichtung in der Weise gesteuert, dass das Blutgefäß die gewünschte Dicke D2 erreicht und behält.

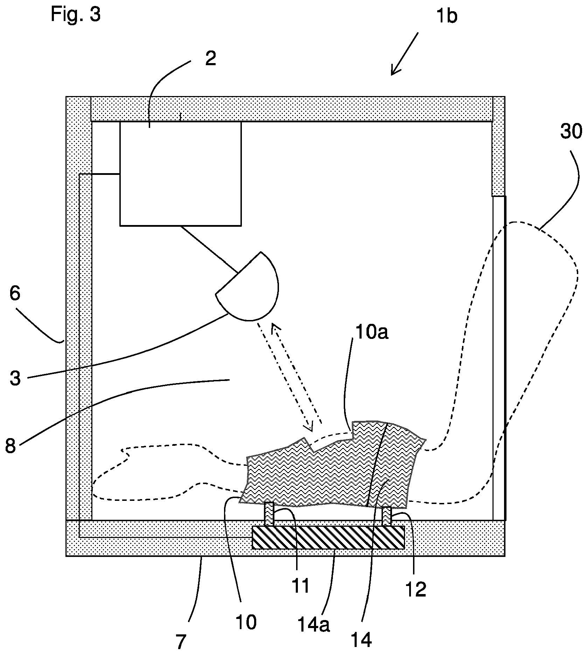

In Figur 3 ist die Erkennungsvorrichtung 1b gezeigt, deren Gefäßmanipulationseinrichtung 14, ebenfalls eine Anpresseinrichtung ist, insbesondere eine um den Arm 30 herumführende und mittels Elektromotor 14a mit Luft befüllbare Schlauchmanschette 10, die an der Auflage 7 fixiert ist oder fixierbar ist. Die Schlauchmanschette 10 weist in proximaler Richtung am Arm einen über den separaten Luftzuführungsabschnitt 12 separat mit Druck beaufschlagbaren Druckbereich 14 auf, in dem ein Anpressdruck gezielt einstellbar ist. Die Schlauchmanschette 10 dient gleichzeitig als Fixiermanschette, indem der Rest der Schlauchmanschette außerhalb des Druckbereich 14 über den Luftzuführungsabschnitt 11 in ausreichender Weise mit Luft befüllbar ist, um den Arm zu fixieren. Der Elektromotor 14a zum Pumpen der Luft wird von der Steuerungseinrichtung in der Weise gesteuert, dass das Blutgefäß die gewünschte Dicke D2 erreicht und behält.

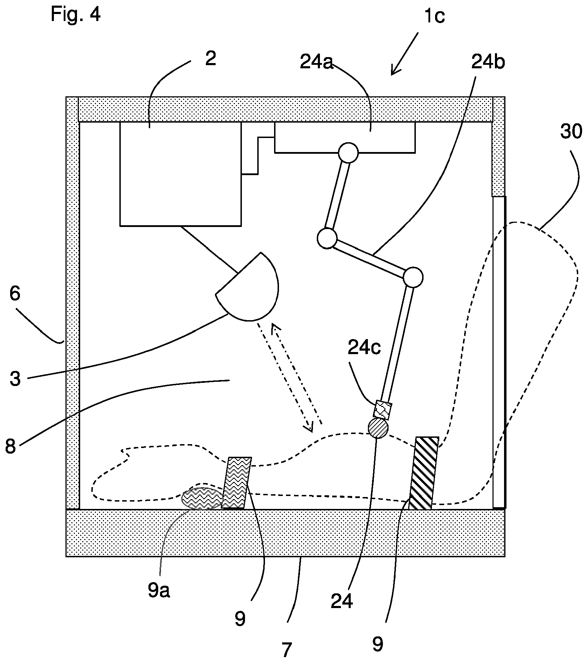

In Figur 4 ist die Erkennungsvorrichtung 1c gezeigt, deren Gefäßmanipulationseinrichtung 4, ebenfalls eine Anpresseinrichtung ist, die eine bewegliche Halteeinrichtung 24b für den Anpresskopf 24 aufweist, insbesondere einen Gelenkarm 24b, der über eine Antriebseinheit 24a des Anpresskopfes gesteuert und bewegt wird. Der Anpresskopf weist eine hier ca. 5 cm² große Kontaktfläche auf, mit der die Haut über dem zu stauenden Blutgefäß kontaktiert und mit der in den Arm gepresst wird. Um die aufgebrachte Kraft zu ermitteln und gegebenenfalls die Aktivität der Gefäßmanipulationseinrichtung 24 zu stoppen oder zu regeln, kann ein Kraftsensor 24c an der Halteeinrichtung 24b vorgesehen sein. Der Antrieb 24a wird von der Steuerungseinrichtung 2 in der Weise gesteuert, dass das Blutgefäß die gewünschte Dicke D2 erreicht und behält.

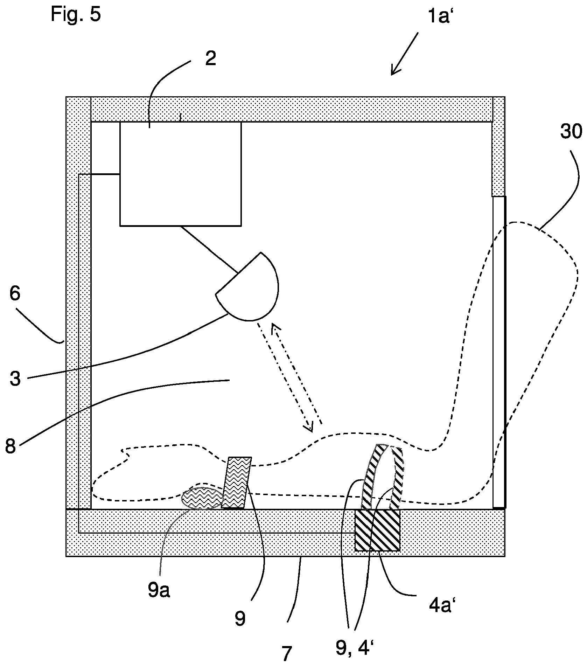

In Figur 5 ist die Erkennungsvorrichtung 1a' gezeigt, deren Gefäßmanipulationseinrichtung 4', ebenfalls eine Anpresseinrichtung ist, die zwei bewegliche Anpress- und Halteklammerarme 4' aufweist, die von einem Elektromotor 4a' in der gewünschten Weise um den Arm 30 gelegt und an diesen Arm gepresst werden. Dabei kann der Gewebeabschnitt, der das Blutgefäß enthält, zwischen den Enden der Halteklammern 4' vom Körperteil weg radial nach außen gepresst werden, wodurch einerseits die gewünschte Blutstauung erreicht wird und andererseits das Blutgefäß zugänglich ist und zusätzlich fixiert wird. Der Elektromotor 4a' wird von der Steuerungseinrichtung 2 in der Weise gesteuert, dass das Blutgefäß die gewünschte Dicke D2 erreicht.

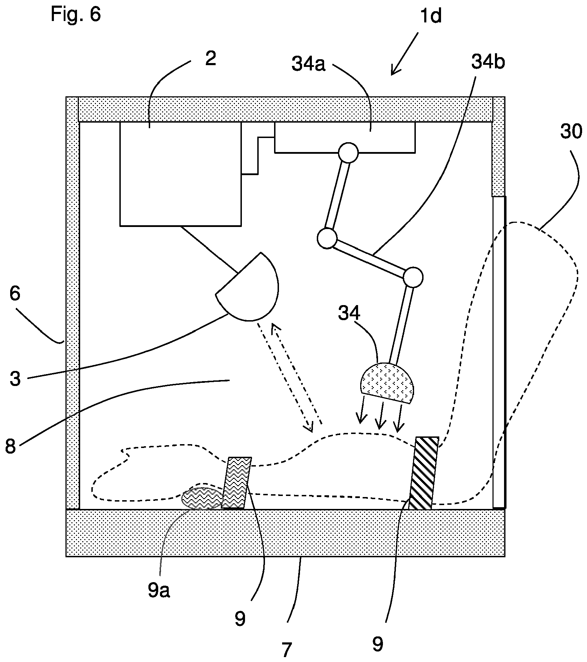

Figur 6 zeigt die Erkennungsvorrichtung 1d, bei der die Gefäßmanipulationseinrichtung als Wärmeübertragungseinrichtung 34 ausgeführt ist. Die Wärmeübertragungseinrichtung 34 ist hier eine an der beweglichen Halteeinrichtung 34b, insbesondere dem Gelenkarm 34b, angebrachte Infrarotlampe, deren Position und/oder Leistung über die Antriebs- und Steuereinheit 34a der Infrarotlampe eingestellt wird (Leistung gemessen in kW). Die Position, Leistung und/oder Kontaktdauer der Wärmeübertragungseinrichtung 34 wird so eingestellt und/oder betrieben, dass das Blutgefäß aufgrund der thermisch erhöhten Durchblutung die gewünschte Dicke D2 erreicht und insbesondere behält.

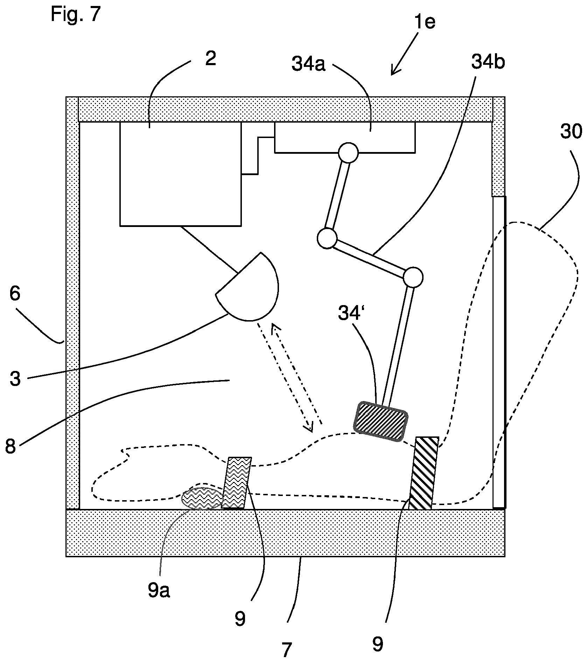

Figur 7 zeigt die Erkennungsvorrichtung 1e, bei der die Gefäßmanipulationseinrichtung als Wärmeübertragungseinrichtung 34' ausgeführt ist. Die Wärmeübertragungseinrichtung 34' ist hier eine an der beweglichen Halteeinrichtung 34b, insbesondere dem Gelenkarm 34b, angebrachtes, die Haut kontaktierendes Wärmeübertragungselement 34' mit integrierter Temperiereinrichtung, z.B. einem Peltierelement. Die Position des Wärmeübertragungselements und/oder die Leistung der Temperiereinrichtung kann über die Antriebs- und Steuereinheit 34a eingestellt werden. Die Position, Leistung und/oder Kontaktdauer der Wärmeübertragungseinrichtung 34' wird so eingestellt und/oder betrieben, dass das Blutgefäß aufgrund der thermisch erhöhten Durchblutung die gewünschte Dicke D2 erreicht und insbesondere behält.

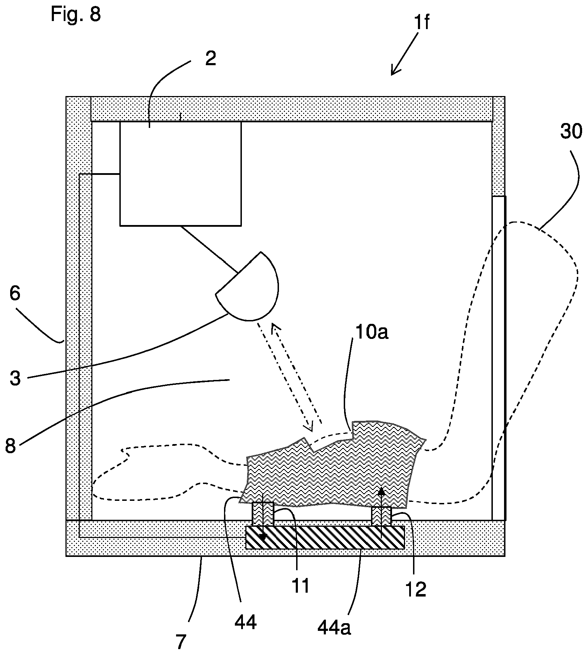

Figur 8 zeigt die Erkennungsvorrichtung 1f, bei der die Gefäßmanipulationseinrichtung als Wärmeübertragungseinrichtung 44 ausgeführt ist. Die Wärmeübertragungseinrichtung 44 ist hier eine von einem Wärmeübertragungsfluid durchströmbare Körperteilmanschette 44, die hier den Arm bis auf das Fenster 10a umgibt. Das fluide Medium, vorzugsweise Wasser, wird in der Steuerung 44a der Armmanschette 44 mittels Temperiereinrichtung, z.B. elektrischer Widerstandseinrichtung, temperiert. Das temperierte Medium wird mittels Pumpe (nicht gezeigt) in der Steuerung 44a über den Zugangsanschluss 12 durch die Armmanschette 44 eingepumpt. In der Schlauchmanschette kann ein Kanalsystem vorgesehen sein, insbesondere ein spiralförmig um den Arm verlaufender Leitungskanal oder mäandernder Leitungskanal. Mittels dem Kanalsystem wird das Medium über die Fläche der Armmanschette 44 verteilt, um den Arm 30 vorliegend gleichmäßig zu erwärmen. Über den Abflussanschluss 11 der Armmanschette 44 tritt das Medium wieder aus und wird zur Zirkulation zurück in die Steuerung 44a geführt. Die Leistung der Temperiereinrichtung kann über die Steuerung 44a eingestellt werden. Die Leistung der Wärmeübertragungseinrichtung 44 wird so eingestellt und/oder betrieben, dass das Blutgefäß aufgrund der thermisch erhöhten Durchblutung die gewünschte Dicke D2 erreicht und insbesondere behält.

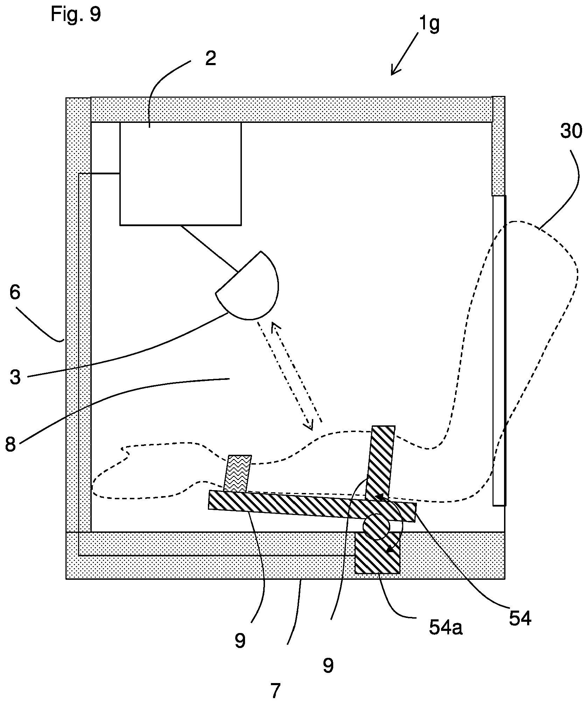

Figur 9 zeigt die Erkennungsvorrichtung 1g, bei der die Gefäßmanipulationseinrichtung 54 als bewegliche Stützeinrichtung zum beweglichen Stützen des Arms ausgeführt ist. Die bewegliche Stützeinrichtung weist eine mittels Gelenk rotierbare Plattform auf, die als Auflagefläche für den Arm dient, an der dieser zudem mittels Fixierbändern 9 fixierbar ist. Die Rotation um eine oder mehrere Achsen wird über den in der Steuerung 54a vorgesehenen Elektromotor von der Steuerungseinrichtung 2 in der Weise gesteuert, dass das im Arm 30 enthaltene und erkannte Blutgefäß die gewünschte Lage erreicht (siehe Fig. 13).

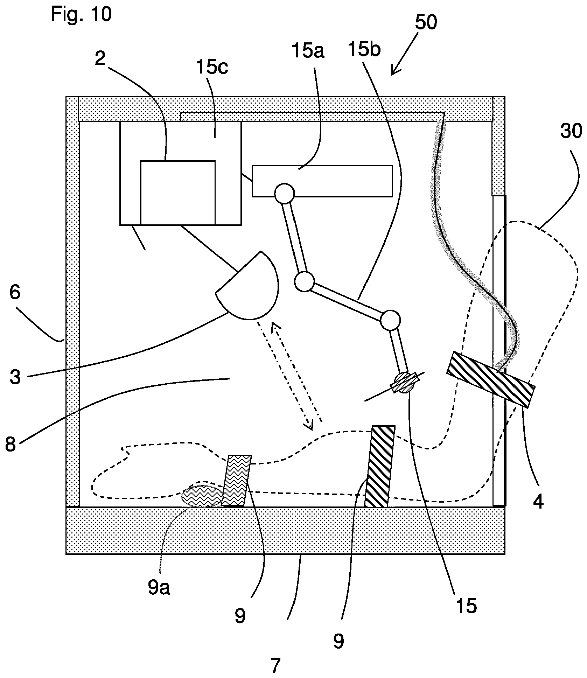

Figur 10 zeigt den erfindungsgemäßen Kanülierautomaten 50, der eine erfindungsgemäße Erkennungsvorrichtung 1 aufweist. Der Kanülierautomat 50 weist einen robotergesteuerten Gelenkarm 15b und einen Motor mit Motorsteuerung 15a auf, dessen Werkzeugkopf 15 eine Kanüle trägt, mit der die automatische Punktion und Kanülierung des mittels der Gefäßstrukturmesseinrichtung 3 erkannten und mittels der Gefäßmanipulationseinrichtung 4 manipulierten Blutgefäßes erfolgt. Die Steuerungseinrichtung 2 des Kanülierautomaten enthält die Steuerungseinrichtung 2 der Erkennungsvorrichtung. Funktionen des Kanülierautomaten und der Erkennungsvorrichtung können durch Programmcode implementiert sein, der vom Prozessor der Steuerungseinrichtung 2 ausgeführt wird.

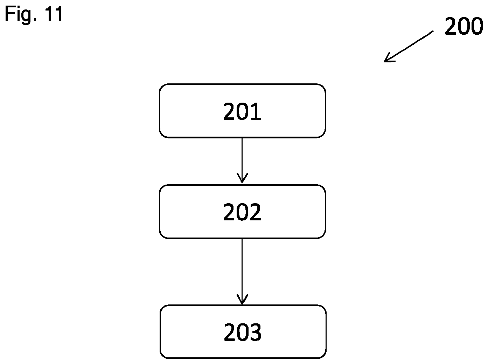

Figur 11 zeigt ein Ausführungsbeispiel des erfindungsgemäßen Verfahrens zum Betreiben einer Erkennungsvorrichtung, aufweisend die Schritte: Schritt 201- Erkennung der Lage und/oder Abmessungen eines Blutgefäßes in einem Behandlungsraum durch Messung von Gefäßstrukturdaten des Blutgefäßes im Behandlungsraum; - optional Schritt 202: Vergleich der Gefäßstrukturdaten mit Vergleichsdaten, die insbesondere vorbestimmte Referenzwerte der Lage und/oder Abmessung des Blutgefäßes enthalten; Schritt 203 - Änderung von Lage und/oder Abmessung des Blutgefäßes mittels einer Gefäßmanipulationseinrichtung, gesteuert durch eine Steuerungseinrichtung 2 in Abhängigkeit von den Gefäßstrukturdaten.

## Patentansprüche

1. Erkennungsvorrichtung (1; 1a; 1a', 1b; 1c; 1d; 1e; 1f; 1g) zur Erkennung und Manipulation eines Blutgefäßes (31) unter der Haut eines Körperteils (30) eines Patienten, aufweisend
ein Behandlungsraum (8) zur Aufnahme des Körperteils,
eine datenverarbeitende Steuerungseinrichtung (2),
eine Gefäßstrukturmesseinrichtung (3) zur Erkennung der Lage und/oder Abmessungen durch Messung von Gefäßstrukturdaten des Blutgefäßes im Behandlungsraum,
eine Gefäßmanipulationseinrichtung (4; 4'; 14; 24; 34; 34'; 44; 54) zur Änderung von Lage und/oder Abmessung des Blutgefäßes,
wobei die Steuerungseinrichtung dazu eingerichtet ist, die Gefäßmanipulationseinrichtung in Abhängigkeit von den Gefäßstrukturdaten zu steuern, **dadurch gekennzeichnet, dass**
die Steuerungseinrichtung dazu eingerichtet ist, aus den Gefäßstrukturdaten zu ermitteln, ob das Blutgefäß eine vorbestimmte Dicke (D2) aufweist, und die Gefäßmanipulationseinrichtung (4; 4'; 14; 24; 34; 34'; 44; 54) derart zu steuern, dass das Blutgefäß die vorbestimmte Dicke (D2) erreicht und zumindest für eine vorbestimmte Zeitspanne behält.

2. Erkennungsvorrichtung gemäß Anspruch 1, wobei die Gefäßmanipulationseinrichtung als Anpresseinrichtung (4; 4'; 14; 24) ausgebildet ist und die Steuerung der Anpresseinrichtung durch Einstellen eines Anpressdrucks erfolgt.

3. Erkennungsvorrichtung gemäß Anspruch 1 oder 2, wobei die Gefäßmanipulationseinrichtung als Wärmeübertragungseinrichtung (34; 34'; 44) ausgebildet ist und die Steuerung der Wärmeübertragungseinrichtung durch Einstellen einer Zeitspanne der Wärmeübertragung und/oder einer Leistung der Wärmeübertragung erfolgt.

4. Erkennungsvorrichtung gemäß Anspruch 1, 2 oder 3, wobei die Gefäßmanipulationseinrichtung als bewegliche Stützeinrichtung (54) zum Stützen des Körperteils (30) im Behandlungsraum (8) ausgebildet ist und die Steuerung der beweglichen Stützeinrichtung durch Ansteuern eines Motors (54a) zur Steuerung der beweglichen Stützeinrichtung erfolgt.

5. Erkennungsvorrichtung gemäß einem der vorangehenden Ansprüche, **dadurch gekennzeichnet, dass** die Gefäßstrukturmesseinrichtung als Bilderfassungseinrichtung ausgebildet ist und eine optische Messung beinhaltet oder auf Messung mittels Ultraschall beruht.

6. Erkennungsvorrichtung gemäß einem der vorangehenden Ansprüche, **dadurch gekennzeichnet, dass** die Steuerungseinrichtung dazu eingerichtet ist, die Gefäßmanipulationseinrichtung in Abhängigkeit von den Gefäßstrukturdaten zu regeln.

7. Erkennungsvorrichtung gemäß einem der vorangehenden Ansprüche, **dadurch gekennzeichnet, dass** die Steuerungseinrichtung dazu eingerichtet ist, die Änderung oder die Regelung der Lage und/oder Abmessungen des Blutgefäßes innerhalb einer vorbestimmten Zeitspanne durchzuführen.

8. Erkennungsvorrichtung gemäß Anspruch 2 und einem der vorangehenden Ansprüche, **dadurch gekennzeichnet, dass** die Anpresseinrichtung einen Kraft- oder Drucksensor aufweist, um den Anpressdruck zu messen.

9. Erkennungsvorrichtung gemäß Anspruch 8, wobei die Steuerungseinrichtung dazu eingerichtet ist, dass ein vorbestimmter oder von der Steuerungseinrichtung ermittelter maximaler Anpressdruck nicht überschritten wird.

10. Kanülierautomat (50) zur automatischen Kanülierung eines erkannten Blutgefäßes eines Körperteils eines Patienten, der eine Erkennungsvorrichtung gemäß einem der vorangehenden Ansprüche aufweist.

11. Verfahren (200) zum Betreiben einer Erkennungsvorrichtung, aufweisend die Schritte:
- Erkennung der Lage und/oder Abmessungen eines Blutgefäßes in einem Behandlungsraum durch Messung von Gefäßstrukturdaten des Blutgefäßes im Behandlungsraum (201);
- optional: Vergleich der Gefäßstrukturdaten mit Vergleichsdaten, die insbesondere vorbestimmte Referenzwerte der Lage und/oder Abmessung des Blutgefäßes enthalten (202);
- Änderung von Lage und/oder Abmessung des Blutgefäßes mittels einer Gefäßmanipulationseinrichtung, gesteuert durch eine Steuerungseinrichtung in Abhängigkeit von den Gefäßstrukturdaten (203), **dadurch gekennzeichnet, dass**
mittels der Steuerungseinrichtung aus den Gefäßstrukturdaten ermittelt wird, ob das Blutgefäß eine vorbestimmte Dicke (D2) aufweist, und mittels der Steuerungseinrichtung die Gefäßmanipulationseinrichtung derart gesteuert wird, dass das Blutgefäß die vorbestimmte Dicke erreicht und zumindest für eine vorbestimmte Zeitdauer behält.

12. Verfahren der Kanülierung eines Blutgefäßes unter der Haut eines Körperteils eines Patienten, aufweisend die Schritte des Verfahrens nach Anspruch 11 und den folgenden Schritt:
- Nach dem Schritt der Änderung von Lage und/oder Abmessung des Blutgefäßes mittels einer Gefäßmanipulationseinrichtung: automatische Kanülierung des manipulierten Blutgefäßes.

## Claims

1. A detection device (1; 1a; 1a', 1b; 1c; 1d; 1e; 1f; 1g) for detecting and manipulating a blood vessel (31) under the skin of a body part (30) of a patient, comprising a treatment room (8) for receiving the body part,
a data processing control device (2),
a vessel structure measuring device (3) for detecting the position and/or dimensions by measuring vessel structure data of the blood vessel in the treatment room,
a vessel manipulation device (4; 4'; 14; 24; 34; 34'; 44; 54) for changing the position and/or dimensions of the blood vessel,
wherein the control device is configured to control the vessel manipulation device in dependence of the vessel structure data, **characterized in that**
the control device is configured to determine from the vessel structure data whether the blood vessel has a predetermined thickness (D2), and to control the vessel manipulation device (4; 4'; 14; 24; 34; 34'; 44; 54) such that the blood vessel reaches the predetermined thickness (D2) and maintains it at least for a predetermined period of time.

2. A detection device according to claim 1, wherein the vessel manipulation device is designed as a pressing device (4; 4'; 14; 24), and the control of the pressing device is performed by setting a contact pressure.

3. A detection device according to claim 1 or 2, wherein the vessel manipulation device is designed as a heat transfer device (34; 34'; 44), and the control of the heat transfer device is performed by setting a time period of heat transfer and/or a power of heat transfer.

4. A detection device according to claim 1, 2 or 3, wherein the vessel manipulation device is designed as a movable support device (54) for supporting the body part (30) in the treatment room (8), and the control of the movable support device is performed by controlling a motor (54a) for controlling the movable support device.

5. A detection device according to one of the preceding claims, **characterized in that** the vessel structure measuring device is designed as an image acquisition device and includes an optical measurement or is based on measurement by means of ultrasound.

6. A detection device according to one of the preceding claims, **characterized in that** the control device is configured to control the vessel manipulation device in dependence of the vessel structure data.

7. A detection device according to one of the preceding claims, **characterized in that** the control device is configured to perform the change or the control of the position and/or dimensions of the blood vessel within a predetermined period of time.

8. A detection device according to claim 2 and one of the preceding claims, **characterized in that** the pressing device comprises a force or pressure sensor to measure the contact pressure.

9. A detection device according to claim 8, wherein the control device is configured such that a predetermined maximum contact pressure or a maximum contact pressure determined by the control device is not exceeded.

10. A cannulation machine (50) for automatically cannulating a detected blood vessel of a body part of a patient, comprising a detection device according to one of the preceding claims.

11. A method (200) for operating a detection device, comprising the steps:
- detecting the position and/or dimensions of a blood vessel in a treatment room by measuring vessel structure data of the blood vessel in the treatment room (201);
- optionally: comparing the vessel structure data with comparison data which in particular include predetermined reference values for the position and/or dimensions of the blood vessel (202);
- changing the position and/or dimensions of the blood vessel by means of a vessel manipulation device, controlled by a control device in dependence of the vessel structure data (203), **characterized in that**
it is determined by means of the control device from the vessel structure data whether the blood vessel has a predetermined thickness (D2), and the vessel manipulation device is controlled by means of the control device such that the blood vessel reaches the predetermined thickness and maintains it at least for a predetermined period of time.

12. A method of cannulating a blood vessel under the skin of a body part of a patient, comprising the steps of the method according to claim 11 and the following step:
- after the step of changing the position and/or dimensions of the blood vessel by means of a vessel manipulation device: automatically cannulating the manipulated blood vessel.

## Revendications

1. Dispositif de détection (1 ; la ; 1a', 1b ; 1c, 1d ; le ; 1f ; 1g) pour la détection et la manipulation d'un vaisseau sanguin (31) sous la peau d'une partie de corps (30) d'un patient, présentant
un espace de traitement (8) pour la réception de la partie de corps,
un système de commande de traitement de données (2),
un système de mesure de structure de vaisseau (3) pour la détection de la position et/ou des dimensions par mesure de données de structure de vaisseau du vaisseau sanguin dans l'espace de traitement,
un système de manipulation de vaisseau (4 ; 4' ; 14 ; 24 ; 34 ; 34' ; 44 ; 54) pour la modification de la position et/ou dimension du vaisseau sanguin,
dans lequel le système de commande est conçu pour commander le système de manipulation de vaisseau en fonction des données de structure de vaisseau, **caractérisé en ce que**
le système de commande est conçu pour déterminer à partir des données de structure de vaisseau si le vaisseau sanguin présente une épaisseur (D2) prédéfinie, et pour commander le système de manipulation de vaisseau (4 ; 4' ; 14 ; 24 ; 34 ; 34' ; 44 ; 54), de telle sorte que le vaisseau sanguin atteint l'épaisseur (D2) prédéfinie et la garde au moins pendant un laps de temps prédéfini.

2. Dispositif de détection selon la revendication 1, dans lequel le système de manipulation de vaisseau est réalisé sous la forme d'un système de serrage (4 ; 4' ; 14 ; 24) et la commande du système de serrage s'effectue par réglage d'une pression de serrage.

3. Dispositif de détection selon la revendication 1 ou 2, dans lequel le système de manipulation de vaisseau est réalisé sous la forme d'un système de transfert de chaleur (34 ; 34' ; 44) et la commande du système de transfert de chaleur s'effectue par réglage d'un laps de temps du transfert de chaleur et/ou d'une puissance du transfert de chaleur.

4. Dispositif de détection selon la revendication 1, 2 ou 3, dans lequel le système de manipulation de vaisseau est réalisé sous la forme d'un système de support (54) mobile pour le support de la partie de corps (30) dans l'espace de traitement (8) et la commande du système de support mobile s'effectue par commande d'un moteur (54a) pour la commande du système de support mobile.

5. Dispositif de détection selon l'une quelconque des revendications précédentes, **caractérisé en ce que** le système de mesure de structure de vaisseau est réalisé sous la forme d'un système d'acquisition d'image et comporte une mesure optique ou repose sur une mesure au moyen d'ultrasons.

6. Dispositif de détection selon l'une quelconque des revendications précédentes, **caractérisé en ce que** le système de commande est conçu pour régler le système de manipulation de vaisseau en fonction des données de structure de vaisseau.

7. Dispositif de détection selon l'une quelconque des revendications précédentes, **caractérisé en ce que** le système de commande est conçu pour réaliser la modification ou le réglage de la position et/ou des dimensions du vaisseau sanguin pendant un laps de temps prédéfini.

8. Dispositif de détection selon la revendication 2 et l'une des revendications précédentes, **caractérisé en ce que** le système de serrage présente un capteur de force ou de pression, afin de mesurer la pression de serrage.

9. Dispositif de détection selon la revendication 8, dans lequel le système de commande est conçu pour qu'une pression de serrage maximale prédéfinie ou déterminée par le système de commande ne soit pas dépassée.

10. Dispositif de canulation automatique (50) pour la canulation automatique d'un vaisseau sanguin détecté d'une partie de corps d'un patient, qui présente un dispositif de détection selon l'une quelconque des revendications précédentes.

11. Procédé (200) pour faire fonctionner un dispositif de détection, présentant les étapes :
- de détection de la position et/ou des dimensions d'un vaisseau sanguin dans un espace de traitement par mesure de données de structure de vaisseau du vaisseau sanguin dans l'espace de traitement (201) ;
- éventuellement : de comparaison des données de structure de vaisseau à des données de comparaison, qui contiennent en particulier des valeurs de référence prédéfinies de la position et/ou dimension du vaisseau sanguin (202) ;
- de modification de la position et/ou dimension du vaisseau sanguin au moyen d'un système de manipulation de vaisseau, commandée par un système de commande en fonction des données de structure de vaisseau (203), **caractérisé en ce que**
au moyen du système de commande, il est déterminé à partir des données de structure de vaisseau si le vaisseau sanguin présente une épaisseur (D2) prédéfinie, et le système de manipulation de vaisseau est commandé au moyen du système de commande de telle sorte que le vaisseau sanguin atteint l'épaisseur prédéfinie et la garde au moins pendant une durée prédéfinie.

12. Procédé de canulation d'un vaisseau sanguin sous la peau d'une partie de corps d'un patient, présentant les étapes du procédé selon la revendication 11 et l'étape suivante :
- après l'étape de modification de la position et/ou dimension du vaisseau sanguin au moyen d'un système de manipulation de vaisseau : canulation automatique du vaisseau sanguin manipulé.
